# EUROPEAN PATENT APPLICATION

(11) **EP 4 548 901 A1**
(43) Date of publication of application: **07.05.2025**
(21) Application number: 23850061.5
(22) Date of filing: 31.07.2023
(51) Int. Cl.: A61K 6/30, A61K 6/60, A61K 6/62, A61K 6/887, C08F 220/10

(54) **CURABLE COMPOSITION PREPARATION KIT, CURABLE COMPOSITION, CURED ARTICLE, AND DENTAL MATERIAL**

(30) Priority: 03.08.2022 JP 2022124314
(71) Applicant: Mitsui Chemicals, Inc., Tokyo 104-0028 (JP); Kulzer GmbH, 63450 Hanau (DE)
(72) Inventor: DANJO, Takahiro, Sodegaura-shi, Chiba 299-0265 (JP); TAKAHASHI, Issei, Sodegaura-shi, Chiba 299-0265 (JP); LECHMANN-DORN, Maria, 63486 Bruchkobel (DE); MEIER, Christoph, 63486 Bruchkobel (DE)
(74) Representative: Hoffmann Eitle
(86) International application number: PCT/JP2023/028027
(87) International publication number: WO 2024/029507

(57) **Abstract**

There is provided a curable composition preparation kit, including: a first agent containing a first monomer; and a second agent containing a second monomer, in which at least one of the first agent or the second agent each independently contains an ascorbic acid-type compound and a compound A containing at least one of an alkoxysilyl group or a hydroxysilyl group.

## Description

### Technical Field

The present disclosure relates to a curable composition preparation kit, a curable composition, a cured product, and a dental material.

### Background Art

In the dental field, synthetic resin moldings and the like are used to repair tooth defects. For example, a curable composition called cement is used as a tooth substitute for repairing large tooth defects. In recent years, the scope of use of cement has expanded.

Porcelain has come to be used as a dental prosthesis from the viewpoint of aesthetics, and adhesiveness to the porcelain is required as well as to the dental tissue.

For polymerization of a curable composition for dental materials such as cement, a photopolymerization initiator, a chemical polymerization initiator, and the like can be used.

For example, as one of general chemical polymerization initiator systems, there is a redox type polymerization initiator in which an oxidizing agent and a reducing agent are combined. As the redox type polymerization initiator, for example, a polymerization initiator system using an organic peroxide as an oxidizing agent and an aromatic amine compound as a reducing agent is known.

Patent Document 1 discloses a two-paste type dental curable composition including a first agent containing a polymerizable monomer (a) having an acidic group, a polymerizable monomer (b) without an acidic group, a polymerization initiator (c), and a filler (d), and a second agent containing a polymerizable monomer (b) without an acidic group, at least one filler (e) selected from the group consisting of a basic glass filler and alumina, a polymerization accelerator (f), and a silane coupling agent (g) represented by a specific structure.

Patent Document 1: International Patent Publication 2019/004391

### SUMMARY OF INVENTION

### Technical Problem

By using the silane coupling agent, adhesiveness of the obtained cured product to ceramics such as porcelain can be improved, but adhesiveness to dentin may deteriorate.

On the other hand, various polymerization initiators that improve adhesiveness to dentin tend to deteriorate adhesiveness to ceramics.

That is, it is difficult to achieve both adhesiveness to ceramics and adhesiveness to dentin, and a cement that achieves both adhesiveness is required.

An object of an embodiment of the present disclosure is to provide a curable composition preparation kit, a curable composition, a cured product, and a dental material capable of obtaining a cured product with excellent adhesiveness to both ceramics and dentin. Solution to Problem

Specific means for solving the problem include the following embodiments.
<1> A curable composition preparation kit, including: a first agent containing a first monomer; and a second agent containing a second monomer, in which at least one of the first agent or the second agent each independently contains an ascorbic acid-type compound and a compound A containing at least one of an alkoxysilyl group or a hydroxysilyl group.
<2> The curable composition preparation kit according to <1>, in which the compound A has a molecular weight of 200 or more.
<3> The curable composition preparation kit according to <1> or <2>, in which the compound A contains at least one selected from a group consisting of an organosilane containing a polymerizable group and an alkoxy group or a hydroxyl group, and an organosiloxane containing a polymerizable group, an alkoxy group or a hydroxyl group, and a siloxane bond.
<3-1> The curable composition preparation kit according to any one of <1> to <3>, in which the compound A contains a compound represented by the following general formula (X).

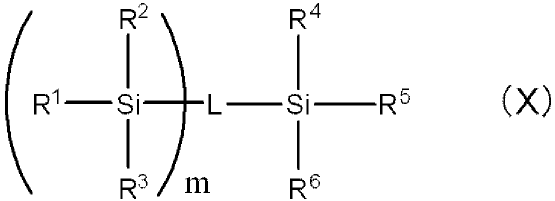

In the general Formula (X), R¹ to R⁶ are each independently a monovalent organic group having a polymerizable group, a monovalent organic group without a polymerizable group, or a hydroxyl group,
m is 0 or 1,
when m is 0, L is a monovalent organic group having a polymerizable group, a monovalent organic group without a polymerizable group, or a hydroxyl group, and
when m is 1, L is an oxygen atom or a structure represented by the following general Formula (Y).

However, when m is 0, at least one of L or R⁴ to R⁶ in the general Formula (X) is a monovalent organic group having a polymerizable group, and at least one is an alkoxy group or a hydroxyl group.

When m is 1, in the general Formula (X) and the general Formula (Y), at least one of R¹ to R⁸ is a monovalent organic group having a polymerizable group, and at least one is an alkoxy group or a hydroxyl group.

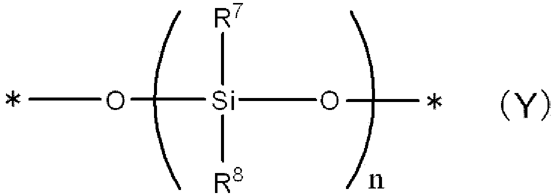

In the general Formula (Y), R⁷ to R⁸ are each independently a monovalent organic group having a polymerizable group, a monovalent organic group without a polymerizable group, a hydroxyl group, or an OSi chain, n is an integer of 1 or more, and * is a bonding position.

The OSi chain is a monovalent organic group having an -O-Si group forming a siloxane bond with adjacent Si in the general Formula (Y).

<4> The curable composition preparation kit according to any one of <1> to <3-1>, in which a content of the compound A is 50% by mass or more with respect to a total amount of the ascorbic acid-type compound and the compound A.

<5> The curable composition preparation kit according to any one of <1> to <4>, in which at least one of the first agent or the second agent each independently contains a transition metal compound and an organic peroxide.

<6> The curable composition preparation kit according to <5>, in which the first agent contains the transition metal compound and the organic peroxide, and the second agent contains the ascorbic acid-type compound and the compound A.

<7> The curable composition preparation kit according to any one of <1> to <6>, in which one of the first monomer and the second monomer contains an acidic monomer.

<8> The curable composition preparation kit according to <7>, in which the first monomer contains the acidic monomer, and the second agent contains the ascorbic acid-type compound and the compound A.

<9> The curable composition preparation kit according to any one of <1> to <8>, in which at least one of the first agent or the second agent contains at least one polymerization accelerator (1) selected from a group consisting of a phosphonite compound, a phosphite compound, and a sulfite compound.

<10> The curable composition preparation kit according to any one of <1> to <9>, in which at least one of the first agent or the second agent contains a polymerization accelerator (2) that is a polymerization accelerator other than at least one polymerization accelerator (1) selected from the group consisting of a phosphonite compound, a phosphite compound, and a sulfite compound.

<11> The curable composition preparation kit according to <1> to <10>, in which at least one of the first agent or the second agent contains a silanol condensing agent.

<12> The curable composition preparation kit according to any one of <1> to <11>, in which the kit is for a dental material.

<13> A curable composition which is a mixture of the first agent and the second agent in the curable composition preparation kit according to any one of <1> to <12>.

<14> A cured product of the curable composition according to <13>.

<15> A dental material including the cured product according to <14>.

### Advantageous Effects of Invention

According to an embodiment of the present disclosure, it is possible to provide a curable composition preparation kit, a curable composition, a cured product, and a dental material capable of obtaining a cured product with excellent adhesiveness to both ceramics and dentin.

### DESCRIPTION OF EMBODIMENTS

In the disclosure, a numerical range indicated using "to" means a range including numerical values described before and after "to" as a minimum value and a maximum value, respectively.

In a numerical range described in stages in the disclosure, an upper limit value or a lower limit value described in a certain numerical range may be replaced with an upper limit value or a lower limit value of another described numerical range in stages. In addition, in the numerical range described in the disclosure, an upper limit value or a lower limit value described in a certain numerical range may be replaced with a value shown in Examples.

In the disclosure, a combination of two or more preferred embodiments is a more preferred embodiment.

In the disclosure, when there are a plurality of kinds of substances corresponding to each component, the amount of each component means the total amount of the plurality of kinds of substances unless otherwise specified.

In the disclosure, "(meth)acryl" means acryl and methacryl, and "(meth)acryloyl" means acryloyl and methacryloyl.

### <<Curable Composition Preparation Kit>>

A curable composition preparation kit of the disclosure includes: a first agent containing a first monomer; and a second agent containing a second monomer, and at least one of the first agent or the second agent each independently contains an ascorbic acid-type compound and a compound A containing at least one of an alkoxysilyl group or a hydroxysilyl group.

"At least one of the first agent and the second agent each independently contains an ascorbic acid-type compound and a compound A containing at least one of an alkoxysilyl group or a hydroxysilyl group" means that at least one of the first agent or the second agent contains an ascorbic acid-type compound, and at least one of the first agent or the second agent contains the compound A.

In a case in which the compound A is contained in both the first agent and the second agent, the compound A contained in the first agent and the compound A contained in the second agent may be the same as or different from each other.

The same applies to the ascorbic acid-type compound.

By using the silane coupling agent, adhesiveness of the obtained cured product to ceramics such as porcelain can be improved, but adhesiveness to dentin may deteriorate. The degree of adhesiveness may often be the degree of no adhesiveness to dentin.

On the other hand, various polymerization initiators that improve adhesiveness to dentin tend to deteriorate adhesiveness to ceramics.

In the curable composition preparation kit of the disclosure, at least one of the first agent or the second agent contains the compound A which is a specific silane coupling agent, but adhesiveness to ceramics can be improved without impairing adhesiveness to dentin.

Ascorbic acid-type compounds in the disclosure can function as a polymerization initiator.

In the curable composition preparation kit of the disclosure, at least one of the first agent or the second agent independently contains an ascorbic acid-type compound and a compound A containing at least one of an alkoxysilyl group or a hydroxysilyl group, and further contains the other configurations described above.

Thus, the curable composition preparation kit of the disclosure can obtain a cured product with excellent adhesiveness to both ceramics and dentin.

### <<Ascorbic Acid-type Compound>>

At least one of the first agent and the second agent contains an ascorbic acid-type compound.

In the disclosure, the ascorbic acid-type compound means ascorbic acid, ascorbic acid derivatives, or salts thereof.

By using an ascorbic acid-type compound, even when at least one of the first agent or the second agent contains the compound A which is a specific silane coupling agent, it is possible to suppress the deterioration in adhesiveness of the cured product to dentin and to obtain a cured product with excellent adhesiveness to both ceramics and dentin.

The ascorbic acid derivative or a salt thereof in the disclosure may include a structure in which at least one of four hydroxyl groups contained in ascorbic acid is substituted with an ether bond bonded to a carbon atom, a structure in which at least one of hydroxyl groups contained in ascorbic acid is substituted with an ester bond bonded to a carbon atom, and the like.

Ascorbic acid-type compounds of the disclosure may be an additive used for a polymerization initiator (polymerization initiator additive), or may be used as a polymerization initiator in combination with other components (for example, transition metal compounds, organic peroxides, and the like).

Examples of the salt of an ascorbic acid derivative in the disclosure include alkali metal salts of ascorbic acid derivatives and alkaline earth metal salts of ascorbic acid derivatives.

More specific examples thereof include sodium salts, potassium salts, magnesium salts, and calcium salts of ascorbic acid derivatives. Among them, calcium salts are preferable from the viewpoint of the balance between polymerizability and adhesiveness.

The ascorbic acid derivative or a salt thereof in the disclosure preferably includes a structure in which at least one of four hydroxyl groups contained in ascorbic acid is substituted with an ether bond bonded to a carbon atom (hereinafter also referred to as an "ether derivative of ascorbic acid") or a structure in which at least one of four hydroxyl groups contained in ascorbic acid is substituted with an ester bond bonded to a carbon atom (hereinafter also referred to as an "ester derivative of ascorbic acid"), and preferably includes an ether derivative of ascorbic acid.

Examples of the ester derivative of ascorbic acid include those having a structure in which at least one of four hydroxyl groups contained in ascorbic acid is formed by a reaction with a carboxylic acid (for example, a carboxylic acid having 2 to 30 carbon atoms).

Suitable examples of the carboxylic acid having 2 to 30 carbon atoms include fatty acids such as caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, bacenic acid, linoleic acid, linoleic acid, linoleelaidic acid, α-linolenic acid, arachidonic acid, eicosapentaenoic acid, erucic acid, and docosahexaenoic acid.

Examples of the ether derivative of ascorbic acid include those having a structure in which at least one of four hydroxyl groups contained in ascorbic acid is formed by reaction with an alkyl halide.

Examples of the alkyl halide include alkyl chloride having 1 to 30 carbon atoms and alkyl bromide having 1 to 30 carbon atoms.

Another example of the ether derivative of ascorbic acid includes one having a structure in which at least two of the four hydroxyl groups contained in ascorbic acid form an ether bond to the same carbon atom (that is, the acetal structure).

The ether derivative of ascorbic acid having an acetal structure is obtained, for example, by reaction of ascorbic acid with an aldehyde.

The ether derivative of ascorbic acid of the disclosure preferably has a structure represented by the following general Formula (A).

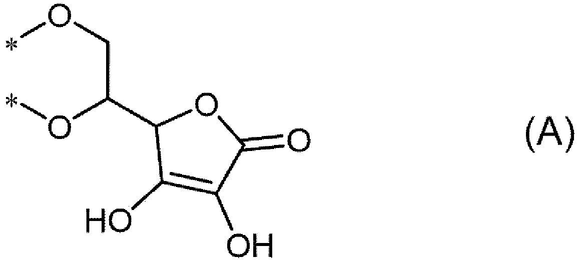

In the general Formula (A), * represents a bonding position with a carbon atom. Two * in the general Formula (A) may be bonded to the same carbon atom to form a 1,3-dioxolane skeleton.

The ascorbic acid derivative in the disclosure preferably contains a compound represented by the following general Formula (B).

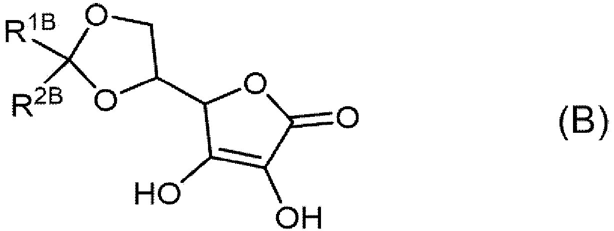

In the general Formula (B), R^{1B} and R^{2B} are each independently a hydrogen atom or a monovalent organic group.

At least one of R^{1B} and R^{2B} is preferably a monovalent organic group.

The monovalent organic group in R^{1B} and R^{2B} is preferably an organic group having 1 to 12 carbon atoms, more preferably an organic group having 1 to 10 carbon atoms, and still more preferably an organic group having 3 to 10 carbon atoms.

In the general Formula (B), one of R^{1B} and R^{2B} may be a hydrogen atom, and the other of R^{1B} and R^{2B} may be a monovalent organic group having 1 to 10 carbon atoms.

The monovalent organic group in R^{1B} and R^{2B} may be an organic group containing an oxygen atom, a nitrogen atom, a sulfur atom, or the like, may be a hydrocarbon group to which an oxygen atom, a nitrogen atom, a sulfur atom, or the like is bonded, or may be a hydrocarbon group.

The salt of the ascorbic acid derivative in the disclosure may be a salt of the compound represented by the general Formula (B), or may be an alkali metal salt or alkaline earth metal salt of the compound represented by the general Formula (B), and from the viewpoint of a balance between polymerizability and adhesiveness, a calcium salt of the compound represented by the general Formula (B) is preferable.

When the salt of the ascorbic acid derivative in the disclosure is a salt of the compound represented by the general Formula (B), R^{1B} and R^{2B} are preferably each independently a hydrogen atom or a monovalent organic group. Further, it is preferable that one of R^{1B} and R^{2B} is a hydrogen atom, and the other of R^{1B} and R^{2B} is a monovalent organic group having 1 to 10 carbon atoms, or R^{1B} and R^{2B} are each independently a monovalent organic group having 1 to 10 carbon atoms.

When one of R^{1B} and R^{2B} is a hydrogen atom and the other of R^{1B} and R^{2B} is a monovalent organic group having 1 to 10 carbon atoms, the other of R^{1B} and R^{2B} is preferably a monovalent organic group having 1 to 5 carbon atoms, more preferably a monovalent organic group having 1 to 3 carbon atoms, further preferably a methyl group, an ethyl group, a propyl group, or an isopropyl group, and particularly preferably an isopropyl group.

When R^{1B} and R^{2B} are each independently a monovalent organic group having 1 to 10 carbon atoms, R^{1B} and R^{2B} are each independently preferably a monovalent organic group having 1 to 5 carbon atoms, more preferably a monovalent organic group having 1 to 3 carbon atoms, further preferably a methyl group, an ethyl group, a propyl group, or an isopropyl group, and particularly preferably a methyl group.

An example of a method for producing an ascorbic acid derivative will be described in detail below.

In this method, ascorbic acid represented by the following general Formula (C) and an aldehyde represented by the following general Formula (D) are subjected to an acetalization reaction (refer to broken lines). R represents, for example, a carbonate group, a hydroxyl group, or the like.

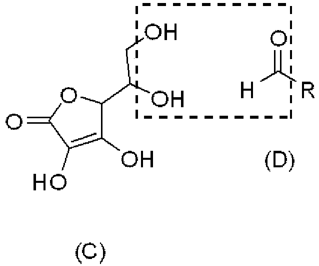

In the general Formula (D), R is the same as R^{1B} or R^{2B} in the general Formula (B).

More specific examples of the aldehyde represented by the general Formula (D) include butanal, isobutanal, hexanal, octanal, and dodecanal. These can be used alone or in combination of two or more kinds thereof.

As reaction conditions for the acetalization reaction, the temperature is, for example, 40°C or higher, preferably 50°C or higher, and more preferably 60°C or higher, and is, for example, 90°C or lower, preferably 80°C or lower, and more preferably 70°C or lower.

The reaction time of the acetalization reaction is, for example, 5 hours or more, preferably 10 hours or more, more preferably 20 hours or more, and is, for example, 100 hours or less, preferably 80 hours or less, and more preferably 60 hours or less.

In the acetalization reaction, a solvent, an acetalization catalyst, and the like can be added as necessary.

Examples of the solvent include amides (for example, dimethylacetamide, dimethylformamide, and the like). These solvents can be used alone or in combination of two or more kinds thereof.

The blending ratio of the solvent is not particularly limited, and is appropriately set according to the purpose and use.

Examples of the acetalization catalyst include known acetalization catalysts such as p-toluenesulfonic acid. These acetalization catalysts can be used alone or in combination of two or more kinds thereof.

The addition ratio of the esterification catalyst is appropriately set according to the purpose and use.

Hereinafter, an example of a method for producing an ascorbic acid derivative alkali metal salt will be described in detail.

In this method, an ascorbic acid derivative represented by the following general Formula (B) is reacted with an alkali metal salt or alkaline earth metal salt represented by the following general Formula (E).

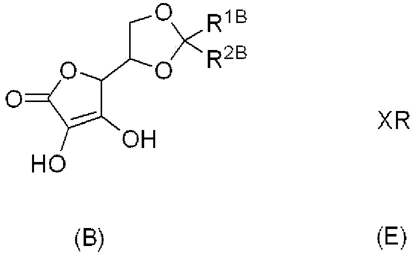

In the general Formula (E), X represents an alkali metal or an alkaline earth metal, and R represents, for example, a carbonate group, a hydroxyl group, or the like.

More specific examples of the alkali metal salt or alkaline earth metal salt represented by the general Formula (E) include sodium hydroxide, sodium hydrogen carbonate, sodium carbonate, calcium hydroxide, and calcium carbonate. These can be used alone or in combination of two or more kinds thereof.

As reaction conditions for the alkali metallization or alkaline earth metallization reaction, the temperature is, for example,-10°C or higher, preferably-5°C or higher, and more preferably 0°C or higher, and is, for example, 100°C or lower, preferably 90°C or lower, and more preferably 80°C or lower.

The reaction time of the alkali metallization or alkaline earth metallization reaction is, for example, 0.1 hours or more, preferably 0.5 hours or more, and more preferably 1 hour or more, and is, for example, 10 hours or less, preferably 8 hours or less, and more preferably 6 hours.

In the alkali metallization or alkaline earth metallization reaction, a solvent or the like can be added as necessary.

Examples of the solvent include alcohols (for example, methanol, ethanol, 2-propanol, butanol, hexanol, and the like), furans (tetrahydrofuran), and ketones (acetone, methyl ethyl ketone, and the like). These solvents can be used alone or in combination of two or more kinds thereof.

The blending ratio of the solvent is not particularly limited, and is appropriately set according to the purpose and use.

### <Compound A>

In the curable composition preparation kit of the disclosure, at least one of the first agent or the second agent contains a compound A containing at least one of an alkoxysilyl group or a hydroxysilyl group.

When the compound A contains at least one of an alkoxysilyl group or a hydroxysilyl group, adhesiveness of the cured product can be improved.

The compound A can be used as a so-called silane coupling agent.

The compound A is not particularly limited as long as it is a compound containing at least one of an alkoxysilyl group or a hydroxysilyl group.

The compound A preferably contains at least one selected from the group consisting of an organosilane containing a polymerizable group and an alkoxy group or a hydroxyl group, and an organosiloxane containing a polymerizable group, an alkoxy group or a hydroxyl group, and a siloxane bond.

The organosilane in the disclosure preferably contains a polymerizable group and an alkoxy group or a hydroxyl group. Preferably, the four bonding sites of the silicon atom in the organosilane are each independently bonded to a monovalent organic group having a polymerizable group, a monovalent organic group without a polymerizable group, or a hydroxyl group, at least one bonding site is bonded to a monovalent organic group having a polymerizable group, and at least one bonding site is bonded to an alkoxy group or a hydroxyl group.

In the monovalent organic group having a polymerizable group, examples of the polymerizable group include a (meth)acryloyl group, an epoxy group, and an allyl group.

Among the above, the polymerizable group preferably includes a (meth)acryloyl group.

The monovalent organic group having a polymerizable group in the organosilane in the disclosure has preferably 3 to 30 carbon atoms, more preferably 4 to 20 carbon atoms, and still more preferably 5 to 15 carbon atoms.

The monovalent organic group having a polymerizable group in the organosilane in the disclosure may be a simple hydrocarbon group, or may contain a urethane bond, an ester bond, an ether bond, or the like.

Examples of the monovalent organic group without a polymerizable group in the organosilane in the disclosure include an optionally substituted alkyl group, an alkoxy group, and the like, and an alkoxy group is preferable.

When the substituent is substituted, examples of the substituent include a hydroxyl group and an amino group.

The monovalent organic group without a polymerizable group in the organosilane in the disclosure has preferably 1 to 10 carbon atoms, more preferably 1 to 7 carbon atoms, and still more preferably 1 to 4 carbon atoms.

The organosiloxane in the disclosure preferably contains a polymerizable group, an alkoxy group or a hydroxyl group, and a siloxane bond (Si-O-Si). Preferably, the remaining six bonding sites of two silicon atoms in one siloxane bond in the organosiloxane, which do not form the siloxane bond, are each independently bonded to an -O-Si group, a monovalent organic group having a polymerizable group, a monovalent organic group without a polymerizable group, or a hydroxyl group.

Preferably, all -O-Si groups contained in the organosiloxane have at least one of the four bonding sites of the Si bonded to an oxygen atom (-O-) that is bonded to an adjacent Si (that is, form a siloxane bond), and have the remaining three bonding sites each independently bonded to an -O-Si group, a monovalent organic group having a polymerizable group, a monovalent organic group without a polymerizable group, or a hydroxyl group. That is, when an -O-Si group is bonded to the remaining six bonding sites of two silicon atoms in one siloxane bond in the organosiloxane, preferably, the remaining three bonding sites in the -O-Si group are each independently bonded to an -O-Si group, a monovalent organic group having a polymerizable group, a monovalent organic group without a polymerizable group, or a hydroxyl group.

In addition, the organosiloxane of the disclosure has a monovalent organic group having at least one polymerizable group and an alkoxy group or a hydroxyl group, but the monovalent organic group having the polymerizable group and the alkoxy group or the hydroxyl group are each bonded to the remaining three bonding sites of Si in the -O-Si group contained in the organosiloxane, and may be bonded to Si in the same-O-Si group or may be bonded to Si in different-O-Si groups.

The organosiloxane in the disclosure has at least one siloxane bond, but may contain an -O-Si group in addition to one siloxane bond. For example, in addition to one siloxane bond, 10 or less -O-Si groups may be included.

Specific embodiments, preferred embodiments, and the like of the monovalent organic group having a polymerizable group and the monovalent organic group without a polymerizable group in the organosiloxane in the disclosure are the same as the specific embodiments, preferred embodiments, and the like of the monovalent organic group having a polymerizable group and the monovalent organic group without a polymerizable group in the organosiloxane.

The compound A preferably contains a compound represented by the following general Formula (X).

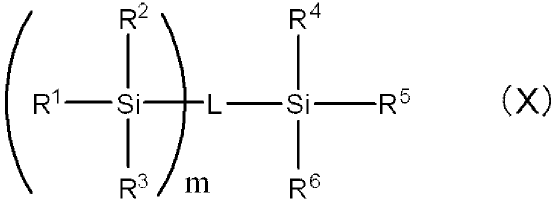

In the general Formula (X), R¹ to R⁶ are each independently a monovalent organic group having a polymerizable group, a monovalent organic group without a polymerizable group, or a hydroxyl group,
m is 0 or 1,
when m is 0, L is a monovalent organic group having a polymerizable group, a monovalent organic group without a polymerizable group, or a hydroxyl group, and
when m is 1, L is an oxygen atom or a structure represented by the following general Formula (Y).

However, when m is 0, at least one of L or R⁴ to R⁶ in the general Formula (X) is a monovalent organic group having a polymerizable group, and at least one is an alkoxy group or a hydroxyl group.

When m is 1, in the general Formula (X) and the general Formula (Y), at least one of R¹ to R⁸ is a monovalent organic group having a polymerizable group, and at least one is an alkoxy group or a hydroxyl group.

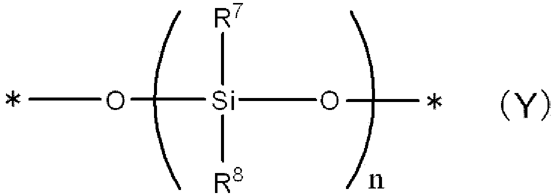

In the general Formula (Y), R⁷ to R⁸ are each independently a monovalent organic group having a polymerizable group, a monovalent organic group without a polymerizable group, a hydroxyl group, or an OSi chain, n is an integer of 1 or more, and * is a bonding position.

The OSi chain is a monovalent organic group having an -O-Si group forming a siloxane bond with adjacent Si in the general Formula (Y).

In the general Formula (X), at least one of R¹ to R³ and at least one of R⁴ to R⁶ are preferably a monovalent organic group having a polymerizable group.

In the general Formula (X), at least one of R¹ to R³ and at least one of R⁴ to R⁶ are preferably an alkoxy group or a hydroxyl group.

In the monovalent organic group having a polymerizable group in L and R¹ to R⁶ of the general Formula (X), examples of the polymerizable group include a (meth)acryloyl group, an epoxy group, and an allyl group.

Among the above, the polymerizable group preferably includes a (meth)acryloyl group.

The monovalent organic group having a polymerizable group in L and R¹ to R⁶ of the general Formula (X) preferably has 3 to 30 carbon atoms, more preferably 4 to 20 carbon atoms, and still more preferably 5 to 15 carbon atoms.

The monovalent organic group having a polymerizable group in L and R¹ to R⁶ of the general Formula (X) may be a simple hydrocarbon group, but may contain a urethane bond, an ester bond, an ether bond, or the like.

Examples of the monovalent organic group without a polymerizable group in L and R¹ to R⁶ of the general Formula (X) include an optionally substituted alkyl group, an alkoxy group, and the like, and an alkoxy group is preferable.

When the substituent is substituted, examples of the substituent include a hydroxyl group and an amino group.

The monovalent organic group without a polymerizable group in L and R¹ to R⁶ of the general Formula (X) preferably has 1 to 10 carbon atoms, more preferably 1 to 7 carbon atoms, and still more preferably 1 to 4 carbon atoms.

Specific embodiments, preferred embodiments, and the like of the monovalent organic group having a polymerizable group in R⁷ to R⁸ of general Formula (Y) are the same as specific embodiments, preferred embodiments, and the like of the monovalent organic group having a polymerizable group in general Formula (X).

Specific embodiments, preferred embodiments, and the like of the monovalent organic group without a polymerizable group in R⁷ to R⁸ of the general Formula (Y) are the same as specific embodiments, preferred embodiments, and the like of the monovalent organic group without a polymerizable group in the general Formula (X).

In the general Formula (Y), n is preferably an integer of 1 to 8, more preferably an integer of 1 to 6, and still more preferably an integer of 1 to 3.

In the general Formula (Y), the OSi chain is a monovalent organic group having an - O-Si group (hereinafter also referred to as an "-O-Si group bonded to Si in the general Formula (Y)") that forms a siloxane bond (that is, -Si-O-Si-) with adjacent Si in the general Formula (Y).

The OSi chain may contain an -O-Si group in addition to the -O-Si group bonded to Si in the general Formula (Y). That is, the OSi chain is a chain structure containing one or two or more -O-Si groups.

The OSi chain has a chain structure including 1 or 2 or more -O-Si groups, preferably 1 to 10, more preferably 1 to 5, and still more preferably 1 to 3.

The OSi chain may have a structure in which siloxane bonds (that is, -Si-O-Si-) are repeatedly continuous.

Examples of the case where the chain structure is linear include a case where the siloxane bond is continuous in a linear form. Similarly, examples of the case where the chain structure is a branched chain include a case where the chain is a branched chain in which the siloxane bond is branched in the middle.

In the OSi chain, preferably, one of the four bonding sites of Si in the -O-Si group bonded to Si in the general Formula (Y) is bonded to an oxygen atom (-O-) bonded to Si in the general Formula (Y), and the remaining three bonding sites are each independently bonded to an -O-Si group (that is, a group forming a siloxane bond), a monovalent organic group having a polymerizable group, a monovalent organic group without a polymerizable group, or a hydroxyl group.

Preferably, in all -O-Si groups (including an -O-Si group bonded to Si in the general Formula (Y)) contained in the OSi chain, at least one of the four bonding sites of the Si is bonded to an oxygen atom (-O-) bonded to adjacent Si, and the remaining three bonding sites are each independently bonded to an -O-Si group (that is, a group forming a siloxane bond), a monovalent organic group having a polymerizable group, a monovalent organic group without a polymerizable group, or a hydroxyl group. That is, for example, when an -O-Si group (that is, a group forming a siloxane bond) is bonded to the four bonding sites of Si in the -O-Si group bonded to Si in the general Formula (Y), preferably, also in the -O-Si group, at least one of the four bonding sites of the Si is bonded to an oxygen atom (-O-) bonded to adjacent Si, and the remaining three bonding sites are each independently bonded to an -O-Si structure (that is, a group forming a siloxane bond), a monovalent organic group having a polymerizable group, a monovalent organic group without a polymerizable group, or a hydroxyl group.

In all -O-Si groups (including an -O-Si group bonded to Si in the general Formula (Y)) contained in the OSi chain, specific embodiments, preferred embodiments, and the like of the monovalent organic group having a polymerizable group bonded to Si and the monovalent organic group without a polymerizable group are the same as each of the specific embodiments, preferred embodiments, and the like of the monovalent organic group having a polymerizable group and the monovalent organic group without a polymerizable group in the general Formula (X).

The content of the compound A is preferably 50% by mass or more, more preferably 60% by mass or more, and still more preferably 70% by mass or more with respect to the total amount of the ascorbic acid-type compound and the compound A.

The content of the compound A may be 95% by mass or less or 90% by mass or less with respect to the total amount of the ascorbic acid-type compound and the compound A.

The content of the compound A is more preferably 1.0% by mass or more, and still more preferably 1.5% by mass or more with respect to the total mass of the curable composition to be prepared.

The content of the compound A is more preferably 12.0% by mass or less, still more preferably 9.0% by mass or more, and particularly preferably 6.0% by mass or less with respect to the total mass of the curable composition to be prepared.

The molecular weight of the compound A is preferably 100 or more, more preferably 150 or more, and still more preferably 200 or more.

The molecular weight of the compound A is preferably 3,000 or less, more preferably 2,000 or less, and still more preferably 1,000 or less.

The compound A may have a molecular weight of 200 to 1,000.

When m in the general Formula (X) is 1, the compound A may be an organosiloxane.

In this case, examples of the method for producing the compound A in the disclosure include a method using an organosilane.

As a specific example of the organosilane, it is preferable to contain 3-acryloxypropylmethoxysilane or 3-methacryloxypropylmethoxysilane from the viewpoint that the obtained compound A is copolymerized with a polymerizable monomer and cured, and from the viewpoint of improving adhesiveness to a ceramic material.

The polymerization by hydrolysis and condensation of the organosilane may be performed by a known method.

For example, the polymerization is generally performed without a solvent, but may be performed in the presence of an organic solvent that dissolves the organosilane used in the reaction.

Polymerization by hydrolysis and condensation may be performed by charging an acid or a base serving as a catalyst of a hydrolysis reaction and water into the above-mentioned organosilane liquid (including a mixed liquid of a plurality of types of organosilanes) or a solution thereof.

The synthesis of the compound A in the disclosure may be performed by appropriately setting conditions according to the target compound A. For example, 0.01 mol to 0.2 mol of hydrochloric acid is added dropwise under stirring with respect to 1 mol of an organosilane at normal temperature, the temperature is raised to about 50°C to 80°C, and hydrolysis condensation is performed for a predetermined period of time (for example, about 2 hours to 72 hours). This is heated to 60°C to 120°C, and the by-produced alcohol is distilled off under normal pressure and concentrated, then filtered, and thus the compound A can be obtained.

The polymerization degree may be adjusted by, for example, a heating temperature, a heating time, an amount of a hydrolysis condensation catalyst (acid or base), or the like. The obtained compound A can be confirmed by analysis of a molecular weight by LC/MS (liquid chromatography mass spectrometry), gel filtration chromatography, or the like.

For the synthesis of the compound A, International Patent Publication 2021/095367 and the like may be referred to.

As the compound A in the disclosure, a commercially available product can also be used. Examples thereof include compounds having an epoxy group, such as KR-517 (manufactured by Shin-Etsu Chemical Co., Ltd.), X-24-9590 (manufactured by Shin-Etsu Chemical Co., Ltd), and KR-516 (manufactured by Shin-Etsu Chemical Co., Ltd.); compounds having a vinyl group, such as KR-511 (manufactured by Shin-Etsu Chemical Co., Ltd.); compounds having a methacrylic group, such as KR-513 (manufactured by Shin-Etsu Chemical Co., Ltd.); and compounds having an acrylic group, such as X-40-9296 (manufactured by Shin-Etsu Chemical Co., Ltd.).

At least one of the first agent and the second agent preferably each independently contains a transition metal compound and an organic peroxide.

"At least one of the first agent and the second agent each independently contains a transition metal compound and an organic peroxide" means that at least one of the first agent or the second agent contains a transition metal compound, and at least one of the first agent or the second agent contains an organic peroxide.

When the transition metal compound is contained in both the first agent and the second agent, the transition metal compound contained in the first agent and the transition metal compound contained in the second agent may be the same or different.

The same applies to the organic peroxide.

More preferably, the first agent contains a transition metal compound and an organic peroxide, and the second agent contains the ascorbic acid-type compound and the compound A.

### <Transition Metal Compound>

As the transition metal compound, a compound soluble in a monomer component in a curable composition preparation kit to be described later is preferable.

Examples of the transition metal compound include a copper compound, a vanadium compound, a molybdenum compound, a scandium compound, a titanium compound, a chromium compound, a manganese compound, an iron compound, a cobalt compound, and a nickel compound.

Among the above, the transition metal compound preferably contains at least one of a copper compound or a vanadium compound, and more preferably contains a copper compound.

Examples of the copper compound include copper acetate, copper isobutyrate, copper gluconate, copper citrate, copper phthalate, copper tartrate, copper oleate, copper octylate, copper octenate, copper naphthenate, copper methacrylate, and copper 4-cyclohexylbutyrate as copper carboxylate; acetylacetone copper, trifluoroacetylacetone copper, hexafluoroacetylacetone copper, 2,2,6,6-tetramethyl-3,5-heptanedionato copper, and benzoylacetone copper as β-diketone copper; ethyl copper acetoacetate as β-ketoester copper; copper methoxide, copper ethoxide, copper isopropoxide, copper 2-(2-butoxyethoxy)ethoxide, and copper 2-(2-methoxyethoxy)ethoxide as copper alkoxide; copper dimethyldithiocarbamate as copper dithiocarbamate; copper nitrate as a salt of copper and an inorganic acid; and copper chloride.

These can be used alone or in combination of two or more kinds as appropriate.

Among them, copper carboxylate, β-diketone copper, and β-ketoester copper are preferable, and copper acetate and acetylacetone copper are more preferable from the viewpoint of solubility and reactivity in monomers.

Examples of the vanadium compound include vanadyl acetylacetonate, vanadium naphthenate(III), vanadyl stearate, vanadium benzoyl acetonate, bis(maltrate)oxovanadium(IV), and oxobis(1-phenyl-1,3-butanedionate)vanadium(IV).

In the curable composition preparation kit of the disclosure, the content of the transition metal compound is preferably 0.00005 parts by mass to 0.1 parts by mass, more preferably 0.0001 parts by mass to 0.05 parts by mass, and still more preferably 0.001 parts by mass to 0.03 parts by mass with respect to total 100 parts by mass of the first agent and the second agent.

### <Organic Peroxide>

The organic peroxide is not particularly limited, and a known organic peroxide can be used. Typical examples of the organic peroxide include hydroperoxide, peroxyester, ketone peroxide, peroxyketal, dialkyl peroxide, diacyl peroxide, and peroxydicarbonate. Among them, hydroperoxide is preferable because the fluctuation in operable time is small even when the curable composition is provided as a dispensing type and stored for a long period of time. The organic peroxide may be used alone or in combination of two or more kinds thereof.

More specific examples of the hydroperoxide include cumene hydroperoxide, t-butyl hydroperoxide, t-hexyl hydroperoxide, p-menthane hydroperoxide, diisopropylbenzene hydroperoxide, 1,1,3,3-tetramethylbutyl hydroperoxide, and t-amyl hydroperoxide.

The peroxy ester can be used without any limitation as long as the peroxy ester contains an acyl group on one side of a peroxy group (-OO- group) and a hydrocarbon group (or a group similar thereto) on the other side. Specific examples thereof include α,α-bis(neodecanoylperoxy)diisopropylbenzene, cumyl peroxyneodecanoate, 1,1,3,3-tetramethylbutyl peroxyneodecanoate, 1-cyclohexyl-1-methylethyl peroxyneodecanoate, t-hexyl peroxyneodecanoate, t-butyl peroxyneodecanoate, t-hexyl peroxypivalate, t-butyl peroxypivalate, 1,1,3,3-tetramethylbutyl peroxy-2-ethylhexanoate, 2,5-dimethyl-2,5-bis(2-ethylhexanoylperoxy)hexane, 1-cyclohexyl-1-methylethyl peroxy-2-ethylhexanoate, t-hexyl peroxy-2-ethylhexanoate, t-butyl peroxy-2-ethylhexanoate, t-butyl peroxyisobutyrate, t-hexyl peroxyisopropyl monocarbonate, t-butyl peroxymaleic acid, t-butyl peroxy-3,5,5-trimethylhexanoate, t-butyl peroxylaurate, 2,5-dimethyl-2,5-bis(m-toluoyl peroxy) hexane, t-butyl peroxyisopropyl monocarbonate, t-butyl peroxy-2-ethylhexyl monocarbonate, t-hexyl peroxybenzoate, 2,5-dimethyl-2,5-bis(benzoylperoxy)hexane, t-butyl peroxyacetate, t-butyl peroxy-m-toluoyl benzoate, t-butyl peroxybenzoate, and bis(t-butyl peroxy)isophthalate. These can be used alone or in combination of two or more kinds as appropriate.

Examples of the ketone peroxide include methyl ethyl ketone peroxide, cyclohexano peroxide, methyl cyclohexanone peroxide, methyl acetoacetate peroxide, and acetyl acetone peroxide.

Examples of the peroxyketal include 1,1-bis(t-hexyl peroxy) 3,3,5-trimethylcyclohexane, 1,1-bis(t-hexyl peroxy)cyclohexane, 1,1-bis(t-butyl peroxy)3,3,5-trimethylcyclohexanone, 1,1-bis(t-butyl peroxy)cyclohexane, 1,1-bis(t-butyl peroxy)cyclodecane, 2,2-bis(t-butyl peroxy)butane, n-butyl-4,4-bis(t-butyl peroxy)valerate, and 2,2-bis(4,4-di-t-butyl peroxycyclohexyl)propane.

Examples of the dialkyl peroxide include α,α-bis(t-butyl peroxy)diisopropylbenzene, dicumyl peroxide, 2,5-dimethyl-2,5-bis(t-butyl peroxy)hexane, t-butylcumyl peroxide, di-t-butyl peroxide, and 2,5-dimethyl-2,5-bis(t-butyl peroxy)hexyne-3.

Examples of the diacyl peroxide include isobutyryl peroxide, 2,4-dichlorobenzoyl peroxide, 3,5,5-trimethylhexanoyl peroxide, octanoyl peroxide, lauroyl peroxide, stearyl peroxide, succinic acid peroxide, m-toluoylbenzoyl peroxide, and benzoyl peroxide.

Examples of the peroxydicarbonate include di-n-propyl peroxydicarbonate, diisopropyl peroxydicarbonate, bis(4-t-butylcyclohexyl)peroxydicarbonate, di-2 ethoxyethyl peroxydicarbonate, di-2-ethylhexyl peroxydicarbonate, di-2-methoxybutyl peroxydicarbonate, and di(3-methyl-3-methoxybutyl)peroxydicarbonate.

The content of the organic peroxide is preferably 0.01 parts by mass to 6 parts by mass, more preferably 0.01 parts by mass to 4 parts by mass, and still more preferably 0.05 parts by mass to 3 parts by mass with respect to total 100 parts by mass of the first agent and the second agent.

In the curable composition preparation kit of the disclosure, preferably, the first agent contains a transition metal compound and an organic peroxide, and the second agent contains the ascorbic acid-type compound from the viewpoint of suppressing the reaction between the ascorbic acid-type compound and the transition metal compound or the organic peroxide.

At least one of the first agent and the second agent may each independently contain sulfinic acid-type compounds, amine compounds, and the like.

### (Sulfinic Acid-type Compounds)

In the disclosure, the sulfinic acid-type compounds means sulfinic acid, sulfinic acid derivatives, and salts thereof.

Examples of the aromatic sulfinic acid-type compound include lithium salts such as benzenesulfinic acid, p-toluenesulfinic acid, o-toluenesulfinic acid, ethylbenzenesulfinic acid, decylbenzenesulfinic acid, dodecylbenzenesulfinic acid, 2,4,6-trimethylbenzenesulfinic acid, 2,4,6-triisopropylbenzenesulfinic acid, chlorbenzenesulfinic acid, and naphthalenesulfinic acid, sodium salts, potassium salts, rubidium salts, cesium salts, magnesium salts, calcium salts, strontium salts, iron salts, zinc salts, ammonium salts, tetramethylammonium salts, and tetraethylammonium salts.

### (Amine Compound)

Examples of the amine compound include aromatic amines, aliphatic amines, and heterocyclic amines.

### (Aromatic Amine)

As the aromatic amine, a known aromatic secondary amine, an aromatic tertiary amine, or the like may be used.

Examples of the aromatic secondary amine or aromatic tertiary amine include N,N-bis(2-hydroxyethyl)-3,5-dimethylaniline, diethanol-p-toluidine (DEPT), N,N-bis(2-hydroxyethyl)-3,4-dimethylaniline, N,N-bis(2-hydroxyethyl)-4 ethylaniline, N,N-bis(2-hydroxyethyl)-4 isopropylaniline, N,N-bis(2-hydroxyethyl)-4 t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, and N,N-dimethyl-3,5-di-t-butylaniline.

In the disclosure, the aromatic amine means an aromatic amine that does not correspond to the heterocyclic amine to be described later.

### (Aliphatic Amine)

Examples of the aliphatic amine include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl(meth)crylate, N-methyldiethanolamine di(meth)acrylate, N-ethyldiethanolamine di(meth)acrylate, triethanolamine tri(meth)acrylate, triethanolamine, trimethylamine, triethylamine, and tributylamine.

### (Heterocyclic Amine)

The heterocyclic amine is an amine having a heterocyclic ring.

Examples of the heterocyclic amine include heterocyclic amines capable of functioning as a monodentate, bidentate, or tridentate heterocyclic electron donor ligand.

Specific examples of the heterocyclic amine include heterocyclic amines derived from unsubstituted or substituted heteroarenes such as furan, thiophene, pyrrole, pyridine, bipyridine, picolilimine, γ-pyran, γ-thiopyran, phenanthroline, pyrimidine, bis-pyrimidine, pyrazine, indole, coumarin, thionaphthene, carbazole, dibenzofuran, dibenzothiophene, pyrazole, imidazole, benzimidazole, oxazole, thiazole, bis-thiazole, isoxazole, isothiazole, quinoline, biquinoline, isoquinoline, biisoquinoline, acridine, chroman, phenazine, phenoxazine, phenothiazine, triazine, thianthrene, purine, bismidazole, and bisoxazoline, and salts thereof.

The heterocyclic amine is preferably 1H-benzotriazole (BTA), 5-methyl-1H-benzotriazole, benzimidazole, or the like.

### <Monomer>

In the curable composition preparation kit of the disclosure, the first agent contains a first monomer, and the second agent contains a second monomer.

The first monomer and the second monomer may be the same monomer or different monomers.

As the first monomer and the second monomer, known monomers can be used.

The first monomer and the second monomer may be monomers without an acidic group, or may be monomers containing an acidic group (hereinafter also referred to as an "acidic monomer").

The first monomer and the second monomer preferably contain a monomer without an acidic group.

The monomer is a monomer that undergo polymerization through a radical polymerization reaction by the action of the ascorbic acid-type compound, transition metal compounds, organic peroxides, and the like of the disclosure.

The monomer constituting the monomer in the disclosure is not limited to one type, and may be two or more types. Examples of the monomer without an acidic group include a (meth)acrylate monomer without an acidic group. Examples of the (meth)acrylate monomer without an acidic group include a monofunctional monomer, a bifunctional monomer, and a tri- or higher functional monomer.

In the disclosure, the monomer content (that is, the total amount of the first monomer and the second monomer in the curable composition to be prepared) is preferably 10% by mass to 90% by mass, more preferably 20% by mass to 75% by mass, and still more preferably 30% by mass to 60% by mass with respect to the total mass of the curable composition to be prepared.

Examples of the monofunctional monomer include 2-hydroxyethyl(meth)crylate, 3-hydroxypropyl(meth)acrylate, 2-hydroxypropyl(meth)acrylate, 1,3-dihydroxypropyl(meth)acrylate, and 2,3-dihydroxypropyl(meth)acrylate. Among them, 2-hydroxyethyl methacrylate (HEMA) is preferable.

Examples of the aromatic compound-based bifunctional monomer include 2,2-bis((meth)cryloyloxyphenyl)propane, 2,2-bis[4-(3-(meth)acryloyloxy)-2-hydroxypropoxyphenyl] propane, 2,2-bis(4-(meth)acryloyloxyethoxyphenyl)propane, 2,2-bis(4-(meth)acryloyloxypolyethoxyphenyl)propane, and 2,2-bis(4-(meth)acryloyloxypolypropylene)propane.

Among them, 2,2-bis[4-(3-(methacryloyloxy)-2-hydroxypropoxyphenyl)propane (commonly called "Bis-GMA") and 2,2-bis(4-methacryloyloxypolyethoxyphenyl)propane are preferable.

Examples of the aliphatic compound-based bifunctional monomer include erythritol di(meth)acrylate, sorbitol di(meth)acrylate, mannitol di(meth)acrylate, pentaerythritol di(meth)acrylate, dipentaerythritol di(meth)acrylate, glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, and, 2,2,4-trimethylhexamethylene bis(2-carbamoyloxyethyl)dimethacrylate (UDMA), and 1,2-bis(3-methacryloyloxy-2-hydroxypropyloxy)ethane.

Among them, glycerol dimethacrylate, triethylene glycol dimethacrylate (TEGDMA), 1,6-hexanediol dimethacrylate (HexDMA), neopentyl glycol dimethacrylate (NPG), 2,2,4-trimethylhexamethylene bis(2-carbamoyloxyethyl) dimethacrylate (UDMA), and 1,2-bis(3-methacryloyloxy-2-hydroxypropyloxy)ethane are preferable.

Examples of the tri- or higher functional monomer include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, N,N-(2,2,4-trimethylhexamethylene)bis[2-(aminocarboxy)propane-1,3-diol]tetramethacrylate, and 1,7-diacryloyloxy-2,2,6,6-tetraacryloyloxymethyl-4-oxyheptane.

Any of the above-mentioned monomers may be blended alone, or may be blended in combination of plural kinds thereof.

The content of the monomer without an acidic group is preferably in a range of 10 parts by mass to 100 parts by mass, more preferably in a range of 20 parts by mass to 100 parts by mass, and still more preferably in a range of 50 parts by mass to 100 parts by mass with respect to total 100 parts by mass of the monomer components in the curable composition preparation kit of the disclosure.

When the monomer component in the curable composition preparation kit of the disclosure contains an acidic monomer to be described later, the content of the monomer without an acidic group is preferably 10 parts by mass to 99 parts by mass, more preferably 30 parts by mass to 97 parts by mass, and still more preferably 50 parts by mass to 95 parts by mass with respect to total 100 parts by mass of the monomer components in the curable composition preparation kit of the disclosure.

The first monomer and the second monomer preferably contain a (meth)acrylic monomer (C) having a molecular weight of 100 to 5,000.

The molecular weight of the (meth)acrylic monomer (C) is more preferably 120 to 3,000, still more preferably 150 to 2,000, and particularly preferably 200 to 1,000.

The content of the (meth)acrylic monomer (C) with respect to the total content of the first monomer and the second monomer is preferably 50% by mass or more, more preferably 70% by mass or more, and still more preferably 90% by mass or more.

### <Acidic Monomer>

In the curable composition preparation kit of the disclosure, it is preferable that any one of the first monomer and the second monomer contains an acidic monomer.

More specifically, in the curable composition preparation kit of the disclosure, preferably, either the first monomer in the first agent or the second monomer in the second agent contains an acidic monomer.

Preferably, the first monomer contains an acidic monomer, and the second agent contains the ascorbic acid-type compound and the compound A.

Accordingly, it is possible to prevent the silane coupling agent from reacting under acidic conditions. That is, the time for reacting the silane coupling agent under acidic conditions can be adjusted by mixing the first agent and the second agent at a desired time period to obtain a curable composition.

When at least one of the first agent or the second agent contains an acidic monomer, for example, in a case where the curable composition preparation kit of the disclosure is used for dental applications, it is possible to provide favorable adhesiveness to dental tissue and high adhesiveness to dental prosthetic materials.

From the viewpoint of suppressing the reaction between the ascorbic acid-type compound and acidic monomers, preferably, the first monomer in the first agent contains an acidic monomer, and the second agent contains the ascorbic acid-type compound, and
preferably, the first monomer in the first agent contains an acidic monomer, and the second agent does not contain an acidic monomer and contains the ascorbic acid-type compound.

Examples of the acidic monomer include monomers having at least one acidic group such as a phosphate group, a pyrophosphate group, a thiophosphate group, a phosphonate group, a sulfonic acid group, or a carboxylic acid group, and at least one polymerizable group such as an acryloyl group, a methacryloyl group, a vinyl group, or a styrene group.

The acidic monomer has affinity for an adherend and has a decalcification effect on the dental tissue.

Examples of the phosphoric acid group-containing monomer include (meth)acryloyloxyalkyl dihydrogen phosphate such as 10-(meth)acryloyloxydecyl dihydrogen phosphate (MDP), bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl dihydrogen phosphate, 2-(meth)acryloyloxyethyl phenyl hydrogen phosphate, 2-(meth)acryloyloxyethyl-2-bromoethyl hydrogen phosphate, bis[2-(meth)acryloyloxy-(1-hydroxymethyl)ethyl]hydrogen phosphate, and acid chlorides thereof, alkali metal salts, and ammonium salts thereof.

Examples of the pyrophosphate group-containing monomer include bis[2-(meth)acryloyloxyethyl]pyrophosphate, bis[4-(meth)acryloyloxybutyl]pyrophosphate, and acid chlorides thereof, alkali metal salts, and ammonium salts thereof.

Examples of the thiophosphate group-containing monomer include 2-(meth)cryloyloxyethyl dihydrogen thiophosphate, 3-(meth)acryloyloxypropyl dihydrogen thiophosphate, and acid chlorides thereof, alkali metal salts, and ammonium salts thereof.

Examples of the phosphonic acid group-containing monomer include 2-(meth)acryloyloxyethylphenylphosphonate, 5-(meth)acryloyloxypentyl-3-phosphonopropionate, and acid chlorides thereof, alkali metal salts, and ammonium salts thereof.

Examples of the sulfonic acid group-containing monomer include 2-(meth)acrylamide-2-methylpropane sulfonic acid, styrene sulfonic acid, and 2-sulfoethyl (meth)acrylate.

Examples of the carboxylic acid group-containing monomer include a monomer containing one carboxy group in the molecule and a monomer containing a plurality of carboxy groups in the molecule.

Examples of the monomer having one carboxy group in the molecule include (meth)acrylic acid, N-(meth)acryloylglycine, N-(meth)acryloylaspartic acid, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, p-vinylbenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4 aminosalicylic acid, 2-(meth)acryloyloxyethyl hydrogen succinate, 2-(meth)acryloyloxyethyl hydrogen phthalate, 2-(meth)acryloyloxyethyl hydrogen malate, and acid halides thereof.

Examples of the monomer having a plurality of carboxy groups in the molecule include 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydecane-1,1-dicarboxylic acid, 13-(meth)acryloyloxytridecane-1,1-dicarboxylic acid, 4-(meth)acryloyloxyethyl trimellitate, 4-(meth)acryloyloxyethyl trimellitate anhydride, 4-(meth)acryloyloxybutyl trimellitate, 4-(meth)acryloyloxyhexyl trimellitate, 4-(meth)acryloyloxydecyl trimellitate, 2-(meth)acryloyloxyethyl-3'-(meth)acryloyloxy-2'-(3,4-dicarboxybenzoyloxy)propyl succinate, dihydroxyethyl methacrylate trimethylhexyl dicarbamate, and acid anhydrides or acid halides thereof.

Among the acidic monomers described above, 10-(meth)acryloyloxydecyl dihydrogen phosphate (MDP), 1,3-di(meth)acryloyloxypropyl dihydrogen phosphate, 2-(meth)acryloyloxyethyl dihydrogen phosphate, 4-(meth)acryloyloxyethyl trimellitate anhydride, 4-(meth)acryloyloxyethyl trimellitate, 2-(meth)acrylamide-2-methylpropane sulfonic acid, 11-(meth)acryloyloxyundecane-1,1-dicarboxylic acid, and dihydroxyethyl methacrylate trimethylhexyl dicarbamate are preferable from the viewpoint of high adhesive strength to an adherend.

The acidic monomer may be used singly or in combination of two or more kinds thereof.

The content of the acidic monomer is preferably 1 part by mass to 50 parts by mass, more preferably 3 parts by mass to 40 parts by mass, and still more preferably 5 parts by mass to 30 parts by mass with respect to 100 parts by mass of the total amount of monomer components in the curable composition preparation kit of the disclosure.

When the content of the acidic monomer is 1 part by mass or more, it is easy to obtain high adhesiveness to various adherends.

When the content of the acidic monomer is 50 parts by mass or less, the balance between polymerizability and adhesiveness is easily maintained. The total amount of monomer components means the total amount of acidic monomers and monomers without the acidic groups described above.

As the monomer in the disclosure, for example, monomers described in known documents such as International Patent Publication 2012/157566, International Patent Publication 2015/015220, International Patent Publication 2015/015221, and Japanese Patent Application Laid-Open (JP-A) No. 2016 094482 can be used.

The total content of the ascorbic acid-type compound contained in the first agent and the second agent is preferably 0.1% by mass to 5% by mass, more preferably 0.3% by mass to 3% by mass, and still more preferably 0.5% by mass to 2% by mass with respect to the total mass of the curable composition to be prepared from the viewpoint of adhesiveness.

In the curable composition preparation kit of the disclosure, at least one of the first agent or the second agent may contain at least one polymerization accelerator (1) selected from the group consisting of a phosphonite compound, a phosphite compound, and a sulfite compound.

From the viewpoint that the polymerization accelerator (1) can function as a stabilizer for the ascorbic acid-type compound, the second agent preferably contains the ascorbic acid-type compound and the polymerization accelerator (1).

### <Phosphonite Compound>

The phosphonite compound may be a trivalent organic phosphorus compound in which a carbon atom is bonded to a phosphorus atom. The phosphonite compound contained in the polymerization accelerator (1) of the disclosure preferably has a structure represented by the following general Formula (I).

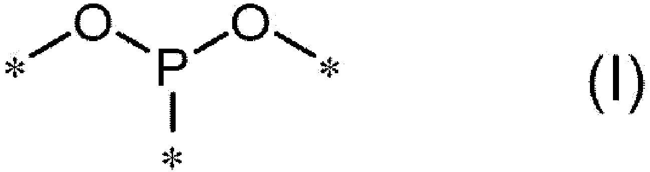

In the general Formula (I), * represents a bonding position to a carbon atom. The three *'s are preferably bonding positions to carbon atoms contained in the hydrocarbon group, and more preferably bonding positions to carbon atoms contained in the benzene ring.

The phosphonite compound may contain one or two or more structures represented by the general Formula (I). The phosphonite compound preferably includes two structures represented by the general Formula (I), and more preferably includes a structure in which two structures represented by the general Formula (I) are bonded via a divalent linking group (preferably, the biphenyl structure).

The phosphonite compound contained in the polymerization accelerator (1) of the disclosure preferably contains a compound represented by the following general Formula (II).

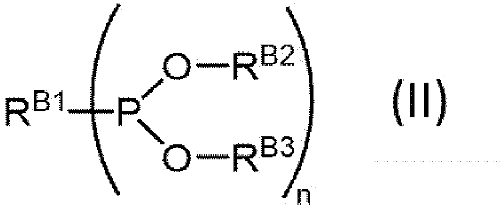

In the general Formula (II), R^{B1} is an n-valent hydrocarbon group, R^{B2} and R^{B3} are each independently a monovalent hydrocarbon group, and n is an integer of 1 or 2.

Examples of the n-valent hydrocarbon group in R^{B1} include an n-valent aliphatic hydrocarbon group, an n-valent alicyclic hydrocarbon group, an n-valent aromatic hydrocarbon group, and a combination of two or more thereof.

The monovalent hydrocarbon group in R^{B1} is preferably an alkyl group, a phenyl group, a biphenyl group, or the like. The hydrogen atom of the phenyl group or the biphenyl group in R^{B1} may be substituted with a substituent such as an alkyl group.

The divalent hydrocarbon group in R^{B1} is preferably an alkylene group, a phenylene group, or a biphenylene group such as a 4,4'-biphenylene group, a 4,3'-biphenylene group, or a 3,3'-biphenylene group. A hydrogen atom contained in the phenylene group or the biphenylene group in R^{B1} may be substituted with a substituent such as an alkyl group.

R^{B2} and R^{B3} are each independently a monovalent hydrocarbon group, and an alkyl group, a phenyl group, or the like is preferable. The hydrogen atom contained in the phenyl group in R^{B2} and R^{B3} may be substituted with an alkyl group such as a tert-butyl group or an n-butyl group.

n is an integer of 1 or 2, preferably 2.

Specific examples of the phosphonite compound contained in the polymerization accelerator (1) of the disclosure include tetrakis(2,4-di-tert-butylphenyl)4,4'-biphenylene-di-phosphonite, tetrakis(2,4-di-tert-butyl-5-methylphenyl)4,4'-biphenylene-di-phosphonite, tetrakis(2,4-di-tert-butylphenyl)4,3'-biphenylene-di-phosphonite, tetrakis(2,4-di-tert-butylphenyl)3,3'-biphenylene-di-phosphonite, tetrakis(2,6-di-tert-butylphenyl)-4,4'-biphenylene-di-phosphonite, tetrakis(2,6-di-tert-butylphenyl)-4,3'-biphenylene-di-phosphonite, tetrakis(2,6-di-tert-butylphenyl)-3,3'-biphenylene-di-phosphonite, bis(2,4-di-tert-butylphenyl)-4-phenyl-phenylphosphonite, bis(2,4-di-tert-butylphenyl)-3-phenyl-phenylphosphonite, bis(2,6-di-n-butylphenyl)-3-phenyl-phenylphosphonite, bis(2,6-di-tert-butylphenyl)-4-phenyl-phenylphosphonite, and bis(2,6-di-tert-butylphenyl)-3-phenyl-phenylphosphonite.

Examples of the phosphite compound include triphenyl phosphite, trisononyl phenyl phosphite, tricresyl phosphite, diphenylmono(2-ethylhexyl)phosphite, diphenyl monodecyl phosphite, diphenylmono(tridecyl)phosphite, and tris(2,4-di-tert-butylphenyl)phosphite.

Examples of the sulfite compound include ethylene sulfite, propylene sulfite, dimethyl sulfite, diethyl sulfite, ethyl methyl sulfite, methyl-n-propyl sulfite, ethyl-n-propyl sulfite, di-n-propyl sulfite, diphenyl sulfite, methyl phenyl sulfite, ethyl sulfite, dibenzyl sulfite, benzyl methyl sulfite, and benzyl ethyl sulfite.

The total content of the polymerization accelerator (1) contained in the first agent and the second agent is preferably 0.1 parts by mass to 5 parts by mass, and more preferably 0.2 parts by mass to 2 parts by mass with respect to 100 parts by mass of the total amount of the first agent and the second agent from the viewpoint of curability.

In the curable composition preparation kit of the disclosure, at least one of the first agent or the second agent may contain a polymerization accelerator (2) that is a polymerization accelerator other than the polymerization accelerator (1).

The polymerization accelerator (2) of the disclosure is not particularly limited, and examples thereof include inorganic salts and thiourea.

Examples of the inorganic salt include sodium sulfite, calcium sulfite, potassium sulfite, potassium nitrate, potassium chloride, potassium sulfate, and sodium chloride.

Examples of the thiourea include acetylthiourea, phenylthiourea, triethylthiourea, tetramethylthiourea, dimethylthiourea, and diphenylthiourea.

The total content of the polymerization accelerator (2) contained in the first agent and the second agent is preferably 0.0001 parts by mass to 1 part by mass, and more preferably 0.001 parts by mass to 0.3 parts by mass with respect to 100 parts by mass of the total amount of the first agent and the second agent from the viewpoint of curability.

From the viewpoint of curability, the total content of the inorganic salt contained in the first agent and the second agent is preferably 0.0001 parts by mass to 0.1 parts by mass, and more preferably 0.001 parts by mass to 0.01 parts by mass with respect to 100 parts by mass of the total amount of the first agent and the second agent.

From the viewpoint of curability, the total content of thiourea contained in the first agent and the second agent is preferably 0.001 parts by mass to 1.0 parts by mass, and more preferably 0.005 parts by mass to 0.5 parts by mass with respect to 100 parts by mass of the total amount of the first agent and the second agent.

In the disclosure, at least one of the first agent or the second agent preferably contains a silanol condensing agent.

In the disclosure, preferably, the first monomer contains a silanol condensing agent, and the second agent contains the ascorbic acid-type compound and the compound A.

The silanol condensing agent can be used without particular limitation.

Examples of the silanol condensing agent include organic titanium compounds such as titanium(IV) tetrabutoxide, titanium(IV) tetrapropoxide, titanium(IV) bis(ethylacetoacetate)diisopropoxide, titanium(IV) bis(ethylhexoxy)bis(2-ethyl-3 hydroxyhexoxide), titanium(IV) acetylacetonate, and titanium(IV) bis(acetylacetonate)diisopropoxide; organic aluminum compounds such as aluminum(III) acetylacetonate, aluminum trisethylacetoacetate, and diisopropoxyaluminum ethylacetoacetate; organotin compounds such as di-n-octyltin dilaurate; phosphoric acid compounds such as phosphoric acid, polyphosphoric acid and salts thereof; and zirconium compounds such as zirconium(IV) tetraacetylacetonate.

One kind of the silanol condensing agent may be used alone, or two or more kinds thereof may be used in combination.

### <Filler>

In the curable composition preparation kit of the disclosure, at least one of the first agent or the second agent may contain a filler, and it is preferable that the first agent and the second agent contain a filler.

The filler may be blended alone, or may be blended in combination of plural kinds. Examples of the filler include an inorganic filler, an organic filler, and a composite filler of an inorganic filler and an organic filler.

Examples of the inorganic filler include fine powders of various glasses (contains silicon dioxide as a main component, and heavy metals and oxides such as boron and aluminum as necessary), various ceramics, diatomaceous earth, kaolin, clay minerals (montmorillonite and the like), active clay, synthetic zeolite, mica, calcium fluoride, ytterbium fluoride, calcium carbonate, calcium phosphate, aluminum sulfate, barium sulfate, calcium sulfate, zirconium dioxide, titanium dioxide, aluminum oxide, boron oxide, barium oxide, lanthanum oxide, strontium oxide, zinc oxide, calcium oxide, lithium oxide, sodium oxide, bismuth oxide, yttrium oxide, calcium phosphate, hydroxyapatite, aluminum hydroxide, sodium fluoride, potassium fluoride, sodium monofluorophosphate, lithium fluoride, and ytterbium fluoride.

A fine powder having a primary particle size of 0.001 µm to 100 µm is preferably used from the viewpoint of adhesive force and handleability.

Specific examples of such an inorganic filler include barium borosilicate glass fine powder (such as 8235, GM27884 and G018-053 (manufactured by Shott)), strontium boroaluminosilicate glass fine powder (such as G018-093 and G018-163 (manufactured by Shott)), lanthanum glass fine powder (such as GM31684 and G018-161 (manufactured by Shott)), fluoroaluminosilicate glass fine powder (such as G018-091 and Schott G018-117 (manufactured by Shott)), zirconium and/or cesium-containing boroaluminosilicate glass fine powder (such as G018-307, G018-308, and G018-310 (manufactured by Shott)), and silica fine powder (such as AEROSIL OX50, AEROSIL 50, AEROSIL 200, AEROSIL 380, AEROSIL R972, AEROSIL 130 (manufactured by Evonik)).

Examples of the organic filler include fine powders of polymethyl methacrylate, polyethyl methacrylate, a polymer of polyfunctional methacrylate, polyamide, polystyrene, polyvinyl chloride, chloroprene rubber, nitrile rubber, and styrene-butadiene rubber.

Examples of the composite filler of an inorganic filler and an organic filler include a composite filler obtained by dispersing an inorganic filler in an organic filler, and an inorganic/organic composite filler obtained by coating an inorganic filler with various polymers.

In order to improve curability, mechanical strength, and handleability, the filler may be used after being surface-treated in advance with a known surface treatment agent such as a silane coupling agent. Examples of the surface treatment agent include vinyltrimethoxysilane, vinyltriethoxysilane, vinyltrichlorosilane, vinyltri(β-methoxyethoxy)silane, γ-methacryloyloxypropyltrimethoxysilane, γ-glycidoxypropyltrimethoxysilane, γ-mercaptopropyltrimethoxysilane, and γ-aminopropyltriethoxysilane.

The total content of the fillers contained in the first agent and the second agent is preferably in a range of 10% by mass to 80% by mass, more preferably in a range of 30% by mass to 80% by mass, and still more preferably in a range of 50% by mass to 75% by mass with respect to the total mass of the curable composition to be prepared.

### (Non-conductive Filler)

In the curable composition preparation kit of the disclosure, at least one of the first agent or the second agent preferably contains a non-conductive filler.

The non-conductive filler means a filler having a resistance value of 1.00 × 10⁻⁴ Ωm or more.

The upper limit of the resistance value of the non-conductive filler is not particularly limited, and may be, for example, 1.00 × 10²⁰ Ωm.

Examples of the material of the non-conductive filler include organic substances such as polyethylene, polystyrene, a phenol resin, an epoxy resin, an acrylic resin, and a benzoguanamine resin; and inorganic substances such as silica (such as dimethylsilylated silica), silicate (borosilicate glass (such as barium borosilicate glass), aluminosilicate glass (such as boroaluminosilicate glass, strontium boroaluminosilicate glass, fluoroaluminosilicate glass, and barium aluminosilicate glass)), ceramic, boron nitride, and barium nitride.

Among the above materials, silica and silicate are preferable as the material of the non-conductive filler, and dimethylsilylated silica and barium aluminosilicate are more preferable.

The first agent contains a non-conductive filler, and the content of the non-conductive filler with respect to the total mass of the first agent is preferably 10% by mass or more, more preferably 20% by mass or more, and still more preferably 30% by mass or more.

When the non-conductive filler is contained in the second agent, the second agent contains the non-conductive filler, and the content of the non-conductive filler with respect to the total mass of the second agent is preferably 10% by mass or more, more preferably 20% by mass or more, and still more preferably 30% by mass or more.

In the curable composition preparation kit of the disclosure, when the non-conductive filler is contained in the first agent, the content of the non-conductive filler with respect to the total mass of the first agent is preferably 10% by mass or more, and when the non-conductive filler is contained in the second agent, the content of the non-conductive filler with respect to the total mass of the second agent is preferably 10% by mass or more.

### <Additive>

In the curable composition preparation kit of the disclosure, at least one of the first agent or the second agent may independently contain additives such as a photopolymerization initiator, a stabilizer (meth)polymerization inhibitor), a colorant, a fluorescent agent, and an ultraviolet absorber.

As the photopolymerization initiator, a known photopolymerization initiator can be used, and examples thereof include camphorquinone (CQ) and ethyl dimethylaminobenzoate (EDB).

In addition, an antimicrobial substance such as cetylpyridinium chloride, benzalkonium chloride, (meth)acryloyloxydecylpyridinium bromide, (meth)acryloyloxyhexadecylpyridinium chloride, (meth)acryloyloxydecylammonium chloride, or triclosan may be blended.

Known dyes and pigments may be blended in the curable composition preparation kit of the disclosure.

The curable composition preparation kit of the disclosure is preferably for a dental material.

Examples of dental materials include, but are not limited to, dental adhesives, dental filling materials, dental sealants (fissure sealants), abutment construction materials, denture base resins, denture base lining materials, crown prosthetic resins (hard crown resins), and dental room temperature polymerizing resins.

The curable composition preparation kit of the disclosure is particularly preferably used as a dental adhesive.

Examples of the dental adhesive material include a dental adhesive resin cement, an orthodontic adhesive material, an adhesive material for fixing an oscillating tooth, an adhesive material for applying a cavity, and a dental bonding material, and a dental adhesive resin cement is preferable.

Examples of the dental filling material include a dental composite resin (including a dental self-adhesive composite resin), a root canal filling material, a temporary sealing agent, and a back layer material.

### <Curable Composition>

The curable composition of the disclosure is a mixture of the first agent and the second agent in the curable composition preparation kit of the disclosure.

That is, the curable composition of the disclosure contains the first monomer, the second monomer, the ascorbic acid-type compound, and the compound A containing at least one of an alkoxysilyl group or a hydroxysilyl group in the disclosure.

The curable composition of the disclosure may further contain components such as the transition metal compound and the organic peroxide described above.

When the curable composition of the disclosure contains the above configuration, excellent adhesiveness to ceramics and adhesiveness to dentin are obtained.

Specific examples and preferred embodiments of the monomer, the ascorbic acid-type compound, the compound A, the transition metal compound, and the organic peroxide in the curable composition are the same as the specific examples and preferred embodiments of the monomer, the ascorbic acid-type compound, the compound A, the transition metal compound, and the organic peroxide described above.

The curable composition of the disclosure preferably further contains a non-conductive filler.

Specific examples, preferred embodiments, and the like of the non-conductive filler in the curable composition are the same as those of the non-conductive filler described above.

The curable composition of the disclosure may include the aforementioned additives.

### <Cured Product>

The cured product of the disclosure is a cured product of the curable composition of the disclosure or a cured product obtained using the curable composition preparation kit of the disclosure.

The cured product of the disclosure can be suitably used as a dental material. That is, the dental material of the disclosure preferably contains the cured product of the disclosure. Examples

Hereinafter, the disclosure will be described in detail with reference to examples, but the disclosure is not limited thereto. The specific numerical values such as the blending ratio (content ratio), the physical property value, and the parameter used in the following description can be substituted for the upper limit values (numerical values defined as "or less" and "less than") or the lower limit values (numerical values defined as "or more" and "more than") of the blending ratio (content ratio), physical property values, parameters, and the like described in the above "DESCRIPTION OF EMBODIMENTS".

Details of the components and the like used in the present example are as follows.

### (Sulfinic Acid-type Compound)

pTSS: sodium salt of p-toluenesulfinic acid
(Aromatic Amine)
DEPT: diethanol-p-toluidine
(Heterocyclic Amine)
BTA: 1H-benzotriazole
(Polymerization Accelerator)
Triphenyl Phosphite: triphenyl phosphite
DitBuPhenyl-biphenyl-phosphonite: tetrakis (2,4-di-tert-butylphenyl)[1,1'-biphenyl]-4,4'-diylbis(phosphonite)
NaCl: sodium chloride
ATH: acetylthiourea

### (Transition Metal Compound: Copper Complex)

Cu(OAc)₂ monohydrate: copper acetate monohydrate

### (Compound A: Silane Coupling Agent)

3-MPS: 3-methacryloxypropyltrimethoxysilane, molecular weight: 248.4, viscosity at 25°C: 2.6 mPa·s 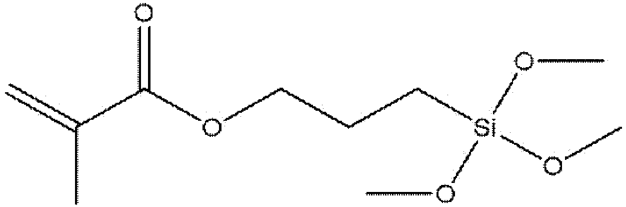
8-MOS: 8-methacryloxyoctyltrimethoxysilane, molecular weight: 318.5, viscosity at 25°C: 5.3 mPa·s 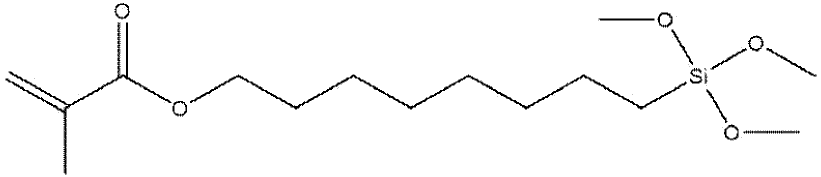
3-APSO: siloxane oligomer of 3-acryloxypropylmethoxysilane, molecular weight distribution: 400 to 800, viscosity at 25°C: 49 mPa·s 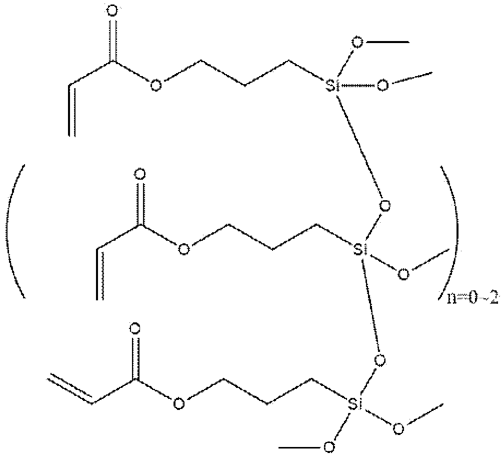
3-MPSO: siloxane oligomer of 3-methacryloxypropylmethoxysilane, molecular weight distribution: 400 to 900, viscosity at 25°C: 29 mPa·s

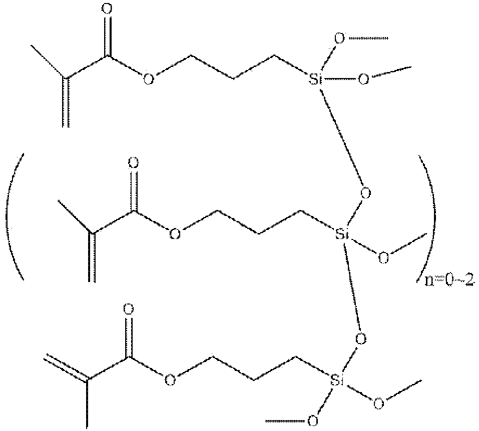

### (Filler)

IS TGF2722A: IS TGF2722A manufactured by Ferro Corporation, 1.0 µm, silane coupling agent treatment amount: 9%
R812: AEROSIL(R)R 812 manufactured by Evonik
AluC: Aeroxide AluC manufactured by EVonik
GM27884 UF1.5 Sil2.3%: GM27884 manufactured by Schott, 1.5 µm, silane coupling agent treatment amount: 2.3%
SG-YBF100WSCMP10: SG-YBF100WSCMP10 manufactured by Sukgyung AT Co., Ltd.

### (Ascorbic Acid-type Compound)

Ascorbic Acid: calcium L-ascorbate dihydrate 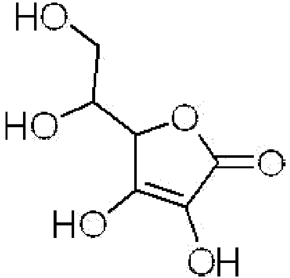
IPD Ascorbic acid: 5-(2,2-dimethyl-1,3-dioxolane-4-yl)-3,4-dihydroxyfuran-2(5H)-one 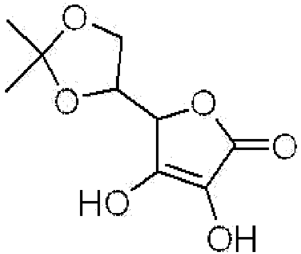
AS6P ester: ascorbic acid palmitoyl ester 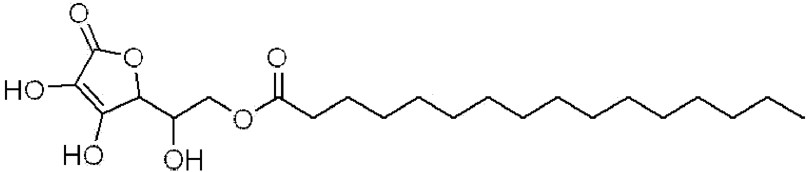
C4isoAcetal Ascorbic acid: 3,4-dihydroxy-5-(2-isopropyl-1,3-dioxolan-4-yl)furan-2(5H)-one 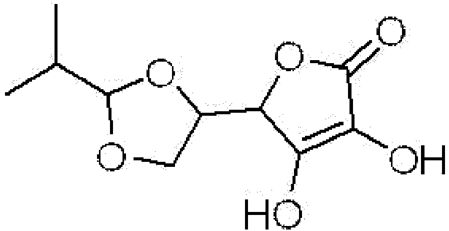
Calcium IPDAA: calcium salt of 5-(2,2-dimethyl-1,3-dioxolane-4-yl)-3,4-dihydroxyfuran-2(5H)-one 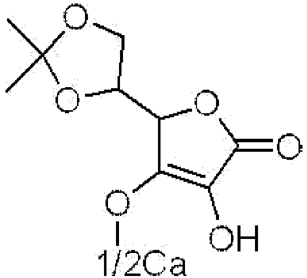
Calcium AS6P ester: calcium salt of ascorbic acid palmitoyl ester 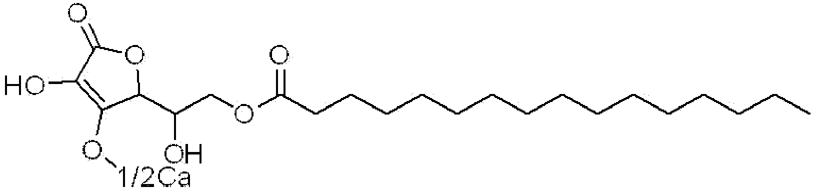
Calcium C4iso AAA: calcium salt of 3,4-dihydroxy-5-(2-isopropyl-1,3-dioxolan-4-yl)furan-2(5H)-one 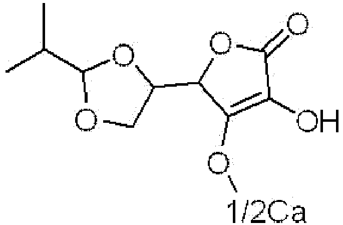
Calcium C12AAA: calcium salt of 3,4-dihydroxy-5-(2-undecyl-1,3-dioxolan-4-yl)furan-2(5H)-one

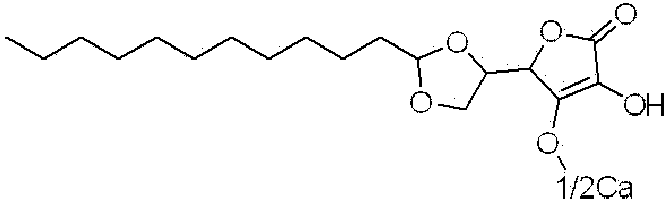

### (Organic Peroxide)

Luperox TAH: tert-amyl hydroperoxide
tBPB: tert-butyl perbenzoate

### (Monomer)

2-HBMA: 2-hydroxybutyl methacrylate
2-HPMA: 2-hydroxypropyl methacrylate
TEGDMA: triethylene glycol dimethacrylate
UDMA: trimethylhexyl diurethane hydroxymethacrylate

### (Acidic Monomer)

MDP: methacryloxydecyl phosphate

### (Photopolymerization Initiator)

CQ: camphorquinone
BEDB: 2-butoxyethyl-4-(dimethylamino)benzoate

### (Stabilizer)

BHT: 2,6-di-tert-butyl-p-cresol

### (Silanol Condensing Agent: Titanium Complex)

Ti(OBu)₄: tetrabutoxy titanate

### [Synthesis Example 1 (C4iso Acetal Ascorbic acid: 3,4-dihydroxy-5-(2-isopropyl-1,3-dioxolan-4-yl)furan-2(5H)-one)]

Ascorbic acid (50.0 g, 284 mmol) and dimethylacetamide (105 mL) as a solvent were charged into an apparatus to which a 300 mL eggplant bottom flask and a Dimroth condenser were connected, the temperature was raised to 60°C, and the solution was stirred uniformly.

Then, isobutanal (20.2 g, 284 mmol) was added, and the solution was stirred uniformly.

Then, p-Toluenesulfonic acid monohydrate (5.7 g, 28 mmol) as an acid catalyst was added, and the solution was stirred uniformly.

After 20 hours, the reaction was stopped, and the temperature was cooled to room temperature.

Thereafter, the obtained reaction product was extracted. That is, an equal amount of distilled water was added to the reaction solution, an equal amount of diethyl ether was further added thereto, and the mixture was extracted and washed with a separatory funnel.

The above extraction was repeated three times, an equal amount of distilled water was added to the obtained organic layer, and the organic layer was washed with a separatory funnel.

The above washing was repeated three times, sodium sulfate was added to the obtained organic layer, and the organic layer was dried and then concentrated under reduced pressure.

The obtained crude product was washed with a hexane/ethyl acetate solvent, and was separated and purified by filtration.

Accordingly, 3,4-dihydroxy-5-(2-isopropyl-1,3-dioxolane-4-yl)furan-2(5H)-one (16 g, 70 mmol, yield 24.6%) was obtained.

The assignment of 3,4-dihydroxy-5-(2-isopropyl-1,3-dioxolan-4-yl)furan-2(5H)-one by ¹H-NMR (CD₃OD) is shown below.

¹H-NMR: δ0.39 (td, 6H, J = 4.5 Hz, 2.2 Hz), 1.19 to 1.30 (m, 1H), 3.39 to 3.79 (m, 3H), 4.10 to 4.19 (m, 2H)

### [Synthesis Example 2 (C12AAA: 3,4-dihydroxy-5-(2-undecyl-1,3-dioxolan-4-yl)furan-2(5H)-one)]

Ascorbic acid (35.224 g, 200 mmol) and dimethylacetamide (180 mL) as a solvent were charged into an apparatus to which a 300 mL eggplant bottom flask and a Dimroth condenser were connected, the temperature was raised to 60°C, and the solution was stirred uniformly.

Then, n-Dodecanal (36.864 g, 200 mmol) was added, and the solution was stirred uniformly.

Then, p-Toluenesulfonic acid monohydrate (3.804 g, 20 mmol) as an acid catalyst was added, and the solution was stirred uniformly.

After 20 hours, the reaction was stopped, and the temperature was cooled to room temperature.

Thereafter, the obtained reaction product was extracted. That is, an equal amount of distilled water was added to the reaction solution, an equal amount of diethyl ether was further added thereto, and the mixture was extracted and washed with a separatory funnel.

The above extraction was repeated three times, an equal amount of distilled water was added to the obtained organic layer, and the organic layer was washed with a separatory funnel.

The above washing was repeated three times, and sodium sulfate was added to the obtained organic layer and dried.

Thereafter, hexane was added, and the precipitated white solid was filtered.

Accordingly, 3,4-dihydroxy-5-(2-undecyl-1,3-dioxolan-4-yl)furan-2(5H)-one (39.5 g, 115.5 mmol, yield 57.7%) was obtained.

The assignment of 3,4-dihydroxy-5-(2-undecyl-1,3-dioxolan-4-yl)furan-2(5H)-one by ¹H-NMR (CD₃OD) is shown below.

1H-NMR: δ0.90 (t, 3H, J = 6.9 Hz), 1.21 to 1.41 (m, 10 H), 1.54 to 1.62(m, 3H), 3.90 to 4.31 (m, 3H), 4.66 (dd, 1H, J = 12.1 Hz, 3.3 Hz), 4.87 to 4.90 (m, 1H)

### [Synthesis Example 3 (Calcium C4isoAAA: 4-hydroxy-2-(2-isopropyl-1,3-dioxolane-4-yl)-5-oxo-2,5-dihydrofuran-3-oleate-calcium)]

A 500 mL eggplant bottom flask was charged with 3,4-dihydroxy-5-(2-isopropyl-1,3-dioxolan-4-yl)furan-2(5H)-one (2.3 g, 10 mmol) and methanol (50 mL) as a solvent, and the solution was stirred uniformly at room temperature.

Further, distilled water (50 mL) was added, and the mixture was stirred uniformly and cooled to 0°C.

Then, calcium hydroxide (5 mmol) was added to the above apparatus, and the mixture was stirred and reacted at 0°C.

After 3 hours, the reaction was stopped, and the mixture was concentrated under reduced pressure.

The obtained crude product was washed with methanol and separated and purified by filtration.

Accordingly, 4-hydroxy-2-(2-isopropyl-1,3-dioxolan-4-yl)-5-oxo-2,5-dihydrofuran-3-olate calcium (1.8 g, 7.2 mmol, 72.2% yield, white solid) was obtained.

The assignment of 4-hydroxy-2-(2-isopropyl-1,3-dioxolan-4-yl)-5-oxo-2,5-dihydrofuran-3-olate calcium by ¹H-NMR (D₂O) is shown below.

¹H-NMR: δ0.72-0.77 (m, 6H), 1.60 to 1.72 (m, 1H), 3.85 to 4.35 (m, 4H), 4.58 to 4.60 (m, 1H)

### [Synthesis Example 4 (Calcium IPDAA: 2-(2,2-dimethyl-1,3-dioxolane-4-yl)-4-hydroxy-5-oxo-2,5-dihydrofuran-3-olate calcium)]

A 500 mL eggplant bottom flask was charged with 5-(2,2-dimethyl-1,3-dioxolane-4-yl)-3,4-dihydroxyfuran-2(5H)-one (2.16 g, 10 mmol) and methanol (50 mL) as a solvent, and the solution was stirred uniformly at room temperature.

Next, distilled water (50 mL) was added to the above apparatus, stirred uniformly, and cooled to 0°C.

Then, calcium hydroxide (5 mmol) was added to the above apparatus, and the mixture was stirred and reacted at 0°C.

After 3 hours, the reaction was stopped, and the mixture was concentrated under reduced pressure.

Thereafter, freeze-drying was carried out, and the mixture was sublimated.

Accordingly, 2-(2,2-dimethyl-1,3-dioxolane-4-yl)-4-hydroxy-5-oxo-2,5-dihydrofuran-3-olate calcium (2.35 g, 10 mmol, 99% yield, white solid) was obtained.

The assignment of 2-(2,2-dimethyl-1,3-dioxolane-4-yl)-4-hydroxy-5-oxo-2,5-dihydrofuran-3-olate calcium by ¹H-NMR (D₂O) is shown below.

¹H-NMR: δ1.24 (s, 3H), 1.26 (s, 3H), 3.99 (dd, 1H, J = 8.9 Hz, 5.7 Hz), 4.14 (dd, 1H, J = 8.8 Hz, 7.1 Hz), 4.32 (d, 1H, J = 2.7 Hz), 4.34 to 4.38 (m, 1H)

### [Synthesis Example 5 (Calcium AS6P ester: calcium salt of palmitoyl ascorbate)]

A 500 mL eggplant bottom flask was charged with palmitoyl ascorbate (2.07 g, 5 mmol) and tetrahydrofuran (100 mL) as a solvent, and the solution was stirred uniformly at room temperature.

Next, distilled water (125 mL) was added to the above apparatus, stirred uniformly, and cooled to 0°C.

Then, calcium hydroxide (2.5 mmol) was added to the above apparatus, and the mixture was stirred and reacted at 0°C.

After 3 hours, the reaction was stopped, and the mixture was concentrated under reduced pressure.

Accordingly, a calcium salt of palmitoyl ascorbate (2.16 g, 5 mmol, 99% yield, white solid) was obtained.

The assignment of the calcium salt of palmitoyl ascorbate by ¹H-NMR (CD₃OD) is shown below.

¹H-NMR: δ0.89 (t, 3H, J = 6.8 Hz), 1.28 (br, 22 H), 1.61 (t, 2H, J = 7.1 Hz), 2.35 (t, 2H, J = 7.4 Hz), 4.10 to 4.26 (m, 2H), 4.36 to 4.36 (m, 2H)

### [Synthesis Example 6 (Calcium C12AAA: calcium salt of 3,4-dihydroxy-5-(2-undecyl-1,3-dioxolan-4-yl)furan-2(5H)-one)]

A 500 mL eggplant bottom flask was charged with 3,4-dihydroxy-5-(2-undecyl-1,3-dioxolan-4-yl)furan-2(5H)-one (3.424 g, 10 mmol) and methanol (100 mL) as a solvent, and the solution was stirred uniformly at room temperature.

Next, distilled water (250 mL) was added to the above apparatus, stirred uniformly, and cooled to 0°C.

Then, calcium hydroxide (10.0 mmol) was added to the above apparatus, and the mixture was stirred and reacted at 0°C.

After 3 hours, the reaction was stopped, and the precipitated white solid was filtered off.

Accordingly, the calcium salt of 3,4-dihydroxy-5-(2-undecyl-1,3-dioxolan-4-yl)furan-2(5H)-one (3.54 g, 9.79 mmol, 97.9% yield, white solid) was obtained.

The assignment of calcium salt of 3,4-dihydroxy-5-(2-undecyl-1,3-dioxolan-4-yl)furan-2(5H)-one by ¹H-NMR (CD₃OD) is shown below.

¹H-NMR: δ0.68 to 0.87 (m, 3H), 1.01 to 1.35 (m, 18 H), 1.42 to 1.67 (m, 2H), 3.63 to 4.36 (m, 4H), 4.97 to 5.02 (m, 1H)

### <Curable Composition Preparation Kit of Examples and Comparative Examples>

Each component shown in Tables 1 to 11 were mixed to prepare the first agent and the second agent in each of Examples and Comparative Examples, thereby obtaining a curable composition preparation kit.

The unit of the content of each component in each Table is % by mass.

### <Evaluation of Adhesiveness>

A cow mandible front tooth was subjected to tooth root cutting and demyelination treatment, and the tooth was placed in a plastic cylindrical container having a diameter of 25 mm and a depth of 25 mm and embedded in an acrylic resin. This is referred to as a bovine tooth adherend. The bovine tooth adherend was polished with water-resistant emery paper (P400) immediately before use, and the smooth surface of the bovine dentin was scraped and used.

Adhesion evaluation was performed according to ISO 16506 using the first agent and the second agent in each of Examples and Comparative Examples described in Tables 1 to 10 separately prepared as test specimens used for measuring adhesive strength.

Specifically, the surface to be coated of the bovine tooth adherend is wiped off with a Kimwipe to remove excessive moisture, and a drop of water is dripped. Next, the first agent and the second agent in each of Examples and Comparative Examples described in Tables 1 to 10 were mixed for 20 seconds, applied in an appropriate amount to the surface to be coated of the cylindrical column cured product (Venus Diamond, manufactured by Kulzer, the surface to be coated was previously polished with water-resistant emery paper P600) of the filler composition prepared in advance, and installed perpendicularly to the bovine tooth adherend prepared as described above. Thereafter, pressure contact was performed with a force of 5 N using a dedicated jig.

After removing the excess of the applied composition, the composition was allowed to stand at 37°C for 40 minutes in a state of being attached to a dedicated jig, then allowed to stand at 37°C for 60 minutes in a state of being detached from the jig, and then allowed to stand in a thermostatic chamber at 37°C for 24 hours, and then the adhesive strength was measured.

The adhesive strength was determined from the shear load when the cylindrical cured product adhered to the bovine tooth adherend was peeled off from the surface by applying a shear load parallel to the bovine tooth adherend and in contact with the surface at a crosshead speed of 1.0 mm/min.

The obtained adhesive strength was evaluated according to the following evaluation criteria, and used as an index of adhesiveness to dentin.

### ~ Evaluation Criteria ~

AA: The adhesive strength was more than 17 MPa.
A: The adhesive strength was more than 14 MPa and 17 MPa or less.
B: The adhesive strength was more than 9 MPa and 14 MPa or less.
C: The adhesive strength was more than 5 MPa and 9 MPa or less.
D: The adhesive strength was 5 MPa or less.
E: No adhesion was observed.

### <Adhesiveness to Ceramics>

A dental feldspar glass ceramic (VITA blocs Mark II manufactured by VITA Zahnfabrik) was installed in a plastic cylindrical container having a diameter of 25 mm and a depth of 25 mm, and embedded in an acrylic resin. This is used as a ceramic adherend. The ceramic adherend was polished with water-resistant emery paper (P600) immediately before use, and the smooth surface of the ceramic was cut out and used.

Adhesion evaluation was performed according to ISO 16506 using the first agent and the second agent in each of Examples and Comparative Examples described in Tables 1 to 10 separately prepared as test specimens used for measuring adhesive strength.

Specifically, equal amounts of the first agent and the second agent in each of Examples and Comparative Examples described in Tables 1 to 10 were weighed, mixed for 20 seconds, applied in an appropriate amount to the adherend surface of the cylindrical column cured product (Venus Diamond, manufactured by Kulzer, the surface to be coated was previously polished with water-resistant emery paper P600) of the filler composition prepared in advance, and installed perpendicularly to the ceramic adherend prepared as described above. Thereafter, pressure contact was performed with a force of 5 N using a dedicated jig.

After removing the excess of the applied composition, in the case of chemical polymerization, the adhesive strength was measured after leaving to stand at 37°C for 40 minutes in a state of being attached to a dedicated jig, then leaving to stand at 37°C for 60 minutes in a state of being detached from the jig, and then leaving to stand in a thermostatic chamber at 37°C for 24 hours.

The adhesive strength was determined from the shear load when the cylindrical cured product adhered to the ceramic adherend was peeled off from the surface by applying a shear load parallel to the ceramic adherend and in contact with the surface at a crosshead speed of 1.0 mm/min.

The obtained adhesive strength was evaluated according to the following evaluation criteria, and used as an index of adhesiveness to feldspar ceramics.

### ~ Evaluation Criteria ~

A: The adhesive strength was more than 17 MPa.
B: The adhesive strength was 13 MPa or more and 17 MPa or less.
C: The adhesive strength was less than 13 MPa.

### <Evaluation 2 of Adhesiveness>

A cow mandible front tooth was subjected to tooth root cutting and demyelination treatment, and the tooth was placed in a plastic cylindrical container having a diameter of 25 mm and a depth of 25 mm and embedded in an acrylic resin. This is referred to as a bovine tooth adherend. The bovine tooth adherend was polished with water-resistant emery paper (P400) immediately before use, and the smooth surface of the bovine dentin was scraped and used.

Adhesion evaluation was performed according to ISO 16506 using the first agent and the second agent in each of Examples described in Table 11 separately prepared as test specimens to be used for measuring adhesive strength.

Specifically, the surface to be coated of the bovine tooth adherend is wiped off with a Kimwipe to remove excessive moisture, and a drop of water is dripped. Next, the first agent and the second agent in each of Examples described in Table 11 were weighed such that the second agent was 1.6 equivalents to the first agent, mixed for 20 seconds, applied in an appropriate amount to the surface to be coated of the cylindrical column cured product (Venus Diamond, manufactured by Kulzer, the surface to be coated was previously polished with water-resistant emery paper P600) of the filler composition prepared in advance, and installed perpendicularly to the bovine tooth adherend prepared as described above. Thereafter, pressure contact was performed with a force of 5 N using a dedicated jig.

After removing the excess of the applied composition, the composition was allowed to stand at 37°C for 40 minutes in a state of being attached to a dedicated jig, then allowed to stand at 37°C for 60 minutes in a state of being detached from the jig, and then allowed to stand in a thermostatic chamber at 37°C for 24 hours, and then the adhesive strength was measured.

The adhesive strength was determined from the shear load when the cylindrical cured product adhered to the bovine tooth adherend was peeled off from the surface by applying a shear load parallel to the bovine tooth adherend and in contact with the surface at a crosshead speed of 1.0 mm/min.

The obtained adhesive strength was evaluated according to the same evaluation criteria as the evaluation criteria of <Evaluation of Adhesiveness>, and used as an index of adhesiveness to dentin.

### <Adhesiveness to Ceramics 2>

A dental feldspar glass ceramic (VITA blocs Mark II manufactured by VITA Zahnfabrik) was installed in a plastic cylindrical container having a diameter of 25 mm and a depth of 25 mm, and embedded in an acrylic resin. This is used as a ceramic adherend. The ceramic adherend was polished with water-resistant emery paper (P600) immediately before use, and the smooth surface of the ceramic was cut out and used.

Adhesion evaluation was performed according to ISO 16506 using the first agent and the second agent in each of Examples described in Table 11 separately prepared as test specimens to be used for measuring adhesive strength.

Specifically, the first agent and the second agent in each of Examples described in Table 11 were weighed such that the second agent was 1.6 equivalents to the first agent, mixed for 20 seconds, applied in an appropriate amount to the surface to be coated of the cylindrical column cured product (Venus Diamond, manufactured by Kulzer, the surface to be coated was previously polished with water-resistant emery paper P600) of the filler composition prepared in advance, and installed perpendicularly to the ceramic adherend prepared as described above. Thereafter, pressure contact was performed with a force of 5 N using a dedicated jig.

After removing the excess of the applied composition, the composition was allowed to stand at 37°C for 40 minutes in a state of being attached to a dedicated jig, then allowed to stand at 37°C for 60 minutes in a state of being detached from the jig, and then allowed to stand in a thermostatic chamber at 37°C for 24 hours, and then the adhesive strength was measured.

The adhesive strength was determined from the shear load when the cylindrical cured product adhered to the ceramic adherend was peeled off from the surface by applying a shear load parallel to the ceramic adherend and in contact with the surface at a crosshead speed of 1.0 mm/min.

The obtained adhesive strength was evaluated according to the same evaluation criteria as the evaluation criteria of <Adhesiveness to Ceramics>, and used as an index of adhesiveness to ceramics.

**[Table 1]**

| | | Example | | | | | | | |
|---|---|---|---|---|---|---|---|---|---|
| | | 1 | | 2 | | 3 | | 4 | |
| | | First agent | Second agent | First agent | Second agent | First agent | Second agent | First agent | Second agent |
| Polymerization accelerator | DitBuPhenyl-biphenyl-phosphonite | | 1.000 | | 1.000 | | 1.000 | | 1.000 |
| | NaCl | | 0.015 | | 0.015 | | 0.015 | | 0.015 |
| Transition metal compound | Cu(OAc)2 monohydrate | 0.020 | | 0.020 | | 0.020 | | 0.020 | |
| Compound A | 3-MPS | | 6.000 | | | | | | |
| | 8-MOS | | | | 6.000 | | | | |
| | 3-APSO | | | | | | 6.000 | | |
| | 3-MPSO | | | | | | | | 6.000 |
| Filler | GM27884 UF1.5 Sil2.3% | | 0.900 | | 0.900 | | 0.900 | | 0.900 |
| | IS TGF2722A | 57.104 | | 57.104 | | 57.104 | | 57.104 | |
| | R812 | 1.318 | 1.381 | 1.318 | 1.381 | 1.318 | 1.381 | 1.318 | 1.381 |
| | SG-YBF100WSCMP10 | | 59.836 | | 59.836 | | 59.836 | | 59.836 |
| Ascorbic acid-type compound | Calcium C4iso AAA | | 1.500 | | 1.500 | | 1.500 | | 1.500 |
| Organic peroxide | Luperox TAH | 2.000 | | 2.000 | | 2.000 | | 2.000 | |
| Monomer | 2-HBMA | 1.757 | 1.841 | 1.757 | 1.841 | 1.757 | 1.841 | 1.757 | 1.841 |
| | MDP | 10.000 | | 10.000 | | 10.000 | | 10.000 | |
| | TEGDMA | 7.907 | 8.285 | 7.907 | 8.285 | 7.907 | 8.285 | 7.907 | 8.285 |
| | UDMA | 19.550 | 18.996 | 19.550 | 18.996 | 19.550 | 18.996 | 19.550 | 18.996 |
| Photopolymerization initiator | BEDB | | 0.100 | | 0.100 | | 0.100 | | 0.100 |
| | CQ | | 0.100 | | 0.100 | | 0.100 | | 0.100 |
| Stabilizer | BHT | 0.044 | 0.046 | 0.044 | 0.046 | 0.044 | 0.046 | 0.044 | 0.046 |
| Silanol condensing agent | Ti(OBu)4 | 0.300 | | 0.300 | | 0.300 | | 0.300 | |
| Adhesiveness evaluation | Dentin (MPa) | B | | AA | | AA | | AA | |
| | Feldspar ceramics (MPa) | A | | A | | A | | A | |

**[Table 2]**

| | | Example | | | |
|---|---|---|---|---|---|
| | | 5 | | 6 | |
| | | First agent | Second agent | First agent | Second agent |
| Polymerization accelerator | DitBuPhenyl-biphenyl-phosphonite | | 1.000 | | 1.000 |
| | NaCl | | 0.015 | | 0.015 |
| Transition metal compound | Cu(OAc)2 monohydrate | 0.020 | | 0.020 | |
| Compound A | 8-MOS | | 6.000 | | 6.000 |
| Filler | GM27884 UF1.5 Sil2.3% | | 0.900 | | 0.900 |
| | IS TGF2722A | 57.104 | | 57.104 | |
| | R812 | 1.318 | 1.381 | 1.318 | 1.381 |
| | SG-YBF100WSCMP10 | | 59.836 | | 59.836 |
| Ascorbic acid-type compound | Calcium AS6P ester | | | | 1.500 |
| | Calcium C4iso AAA | | 1.500 | | |
| Monomer | 2-HBMA | 1.757 | 1.841 | 1.757 | 1.841 |
| | MDP | 10.000 | | 10.000 | |
| | TEGDMA | 7.907 | 8.285 | 7.907 | 8.285 |
| | UDMA | 19.550 | 18.996 | 19.550 | 18.996 |
| Organic peroxide | Luperox TAH | 2.000 | | 2.000 | |
| Photopolymerization initiator | BEDB | | 0.100 | | 0.100 |
| | CQ | | 0.100 | | 0.100 |
| Stabilizer | BHT | 0.044 | 0.046 | 0.044 | 0.046 |
| Silanol condensing agent | Ti(OBu)4 | 0.300 | | 0.300 | |
| Adhesiveness evaluation | Dentin (MPa) | AA | | AA | |
| | Feldspar ceramics (MPa) | A | | A | |

**[Table 3]**

| | | Example | | | | | | | |
|---|---|---|---|---|---|---|---|---|---|
| | | 7 | | 8 | | 9 | | 10 | |
| | | First agent | Second agent | First agent | Second agent | First agent | Second agent | First agent | Second agent |
| Polymerization accelerator | DitBuPhenyl-biphenyl-phosphonite | | 0.500 | | 0.500 | | 0.500 | | 0.500 |
| | NaCl | | 0.015 | | 0.015 | | 0.015 | | 0.015 |
| Transition metal compound | Cu(OAc)2 monohydrate | 0.020 | | 0.020 | | 0.020 | | 0.020 | |
| Compound A | 8-MOS | | 6.000 | | 6.000 | | 6.000 | | 6.000 |
| Filler | GM27884 UF1.5 Sil2.3% | | 61.069 | | 61.069 | | 61.069 | | 61.069 |
| | IS TGF2722A | 57.104 | | 57.104 | | 57.104 | | 57.104 | |
| | R812 | 1.318 | 1.389 | 1.318 | 1.389 | 1.318 | 1.389 | 1.318 | 1.389 |
| Ascorbic acid-type compound | C4iso Acetal Ascorbic acid | | 1.500 | | | | | | |
| | IPD Ascorbic acid | | | | 1.500 | | | | |
| | AS6P ester | | | | | | 1.500 | | |
| | Ascorbic Acid | | | | | | | | 1.500 |
| Monomer | 2-HBMA | 1.757 | 1.851 | 1.757 | 1.851 | 1.757 | 1.851 | 1.757 | 1.851 |
| | MDP | 10.000 | | 10.000 | | 10.000 | | 10.000 | |
| | TEGDMA | 7.907 | 8.331 | 7.907 | 8.331 | 7.907 | 8.331 | 7.907 | 8.331 |
| | UDMA | 19.550 | 19.098 | 19.550 | 19.098 | 19.550 | 19.098 | 19.550 | 19.098 |
| Organic peroxide | Luperox TAH | 2.000 | | 2.000 | | 2.000 | | 2.000 | |
| Photopolymerization initiator | BEDB | | 0.100 | | 0.100 | | 0.100 | | 0.100 |
| | CQ | | 0.100 | | 0.100 | | 0.100 | | 0.100 |
| Stabilizer | BHT | 0.044 | 0.046 | 0.044 | 0.046 | 0.044 | 0.046 | 0.044 | 0.046 |
| Silanol condensing agent | Ti(OBu)4 | 0.300 | | 0.300 | | 0.300 | | 0.300 | |
| Adhesiveness evaluation | Dentin (MPa) | AA | | AA | | A | | B | |
| | Feldspar ceramics (MPa) | A | | A | | A | | A | |

**[Table 4]**

| | | Example | | | |
|---|---|---|---|---|---|
| | | 11 | | 12 | |
| | | First agent | Second agent | First agent | Second agent |
| Polymerization accelerator | DitBuPhenyl-biphenyl-phosphonite | | 0.500 | | 0.500 |
| | NaCl | | 0.015 | | 0.015 |
| Transition metal compound | Cu(OAc)2 monohydrate | 0.020 | | 0.020 | |
| Compound A | 8-MOS | | 6.000 | | 6.000 |
| Filler | AluC | | | | 1.000 |
| | GM27884 UF1.5 Sil2.3% | | 61.069 | | 60.402 |
| | IS TGF2722A | 57.104 | | 57.104 | |
| | R812 | 1.318 | 1.389 | 1.318 | 1.373 |
| Ascorbic acid-type compound | C4iso Acetal Ascorbic acid | | 1.500 | | 1.500 |
| Monomer | 2-HBMA | 1.757 | 1.851 | 1.757 | 1.831 |
| | MDP | 10.000 | | 10.000 | |
| | TEGDMA | 7.907 | 8.331 | 7.907 | 8.239 |
| | UDMA | 19.550 | 19.098 | 19.550 | 18.893 |
| Organic peroxide | Luperox TAH | 2.000 | | 2.000 | |
| Photopolymerization initiator | BEDB | | 0.100 | | 0.100 |
| | CQ | | 0.100 | | 0.100 |
| Stabilizer | BHT | 0.044 | 0.046 | 0.044 | 0.046 |
| Silanol condensing agent | Ti(OBu)4 | 0.300 | | 0.300 | |
| Adhesiveness evaluation | Dentin (MPa) | AA | | AA | |
| | Feldspar ceramics (MPa) | A | | B | |

**[Table 5]**

| | | Example | | Comparative Example | | | |
|---|---|---|---|---|---|---|---|
| | | 13 | | 1 | | 2 | |
| | | First agent | Second agent | First agent | Second agent | First agent | Second agent |
| Polymerization accelerator | DitBuPhenyl-biphenyl-phosphonite | | 1.000 | | | | 1.000 |
| | NaCl | | 0.015 | | | | 0.015 |
| Transition metal compound | Cu(OAc)2 monohydrate | 0.020 | | 0.020 | | 0.020 | |
| Compound A | 8-MOS | | 6.000 | | 6.000 | | 6.000 |
| Filler | AluC | | | | 3.000 | | |
| | GM27884 UF1.5 Sil2.3% | | 0.900 | | 59.036 | | 0.900 |
| | IS TGF2722A | 57.104 | | 58.503 | | 57.104 | |
| | R812 | 1.318 | 1.381 | 1.350 | 1.363 | 1.318 | 1.381 |
| | SG-YBF100WSCMP10 | | 59.836 | | | | 59.836 |
| Ascorbic acid-type compound | Calcium C4iso AAA | | 1.500 | | | | |
| Aromatic amine | DEPT | | | | 0.200 | | |
| Heterocyclic amine | BTA | | | | 1.000 | | |
| Sulfinic acid-type compound | pTSS | | | | 1.000 | | |
| Monomer | 2-HBMA | 1.757 | 1.841 | 1.800 | 1.816 | 1.757 | 1.841 |
| | MDP | 10.000 | | 10.000 | | 10.000 | |
| | TEGDMA | 7.907 | 8.285 | 8.100 | 8.174 | 7.907 | 8.285 |
| | UDMA | 19.550 | 18.996 | 19.981 | 18.165 | 19.550 | 18.996 |
| Organic peroxide | Luperox TAH | 2.000 | | | | 2.000 | |
| | tBPB | | | 0.200 | | | |
| Photopolymerization initiator | BEDB | | 0.100 | | 0.100 | | 0.100 |
| | CQ | | 0.100 | | 0.100 | | 0.100 |
| Stabilizer | BHT | 0.044 | 0.046 | 0.045 | 0.045 | 0.044 | 0.046 |
| Silanol condensing agent | Ti(OBu)4 | 0.300 | | | | 0.300 | |
| Adhesiveness evaluation | Dentin (MPa) | AA | | E | | E | |
| | Feldspar ceramics (MPa) | A | | A | | E | |

**[Table 6]**

| | | Example | | Comparative Example | |
|---|---|---|---|---|---|
| | | 14 | | 3 | |
| | | First agent | Second agent | First agent | Second agent |
| Polymerization accelerator | DitBuPhenyl-biphenyl-phosphonite | | 1.000 | | 1.000 |
| | NaCl | | 0.015 | | 0.015 |
| Transition metal compound | Cu(OAc)2 monohydrate | 0.020 | | 0.020 | |
| Compound A | 8-MOS | | 6.000 | | |
| Filler | GM27884 UF1.5 Sil2.3% | | 0.900 | | 0.900 |
| | IS TGF2722A | 57.104 | | 57.104 | |
| | R812 | 1.318 | 1.381 | 1.318 | 1.473 |
| | SG-YBF100WSCMP10 | | 59.836 | | 63.833 |
| Ascorbic acid-type compound | Calcium C4iso AAA | | 1.500 | | 1.500 |
| Monomer | 2-HBMA | 1.757 | 1.841 | 1.757 | 1.964 |
| | MDP | 10.000 | | 10.000 | |
| | TEGDMA | 7.907 | 8.285 | 7.907 | 8.839 |
| | UDMA | 19.550 | 18.996 | 19.550 | 20.226 |
| Organic peroxide | Luperox TAH | 2.000 | | 2.000 | |
| Photopolymerization initiator | BEDB | | 0.100 | | 0.100 |
| | CQ | | 0.100 | | 0.100 |
| Stabilizer | BHT | 0.044 | 0.046 | 0.044 | 0.049 |
| Silanol condensing agent | Ti(OBu)4 | 0.300 | | 0.300 | |
| Adhesiveness evaluation | Dentin (MPa) | AA | | AA | |
| | Feldspar ceramics (MPa) | A | | C | |

**[Table 7]**

| | | Example | | | | | |
|---|---|---|---|---|---|---|---|
| | | 15 | | 16 | | 17 | |
| | | First agent | Second agent | First agent | Second agent | First agent | Second agent |
| Polymerization accelerator | ATH | | 0.150 | | 0.100 | | 0.050 |
| | Triphenyl Phosphite | | 1.000 | | 1.000 | | 1.000 |
| Transition metal compound | Cu(OAc)2 monohydrate | 0.020 | | 0.020 | | 0.020 | |
| Compound A | 8-MOS | | 6.000 | | 6.000 | | 6.000 |
| Filler | IS TGF2722A | 57.104 | | 57.104 | | 57.104 | |
| | R812 | 1.318 | 1.402 | 1.318 | 1.403 | 1.318 | 1.403 |
| | SG-YBF100WSCMP10 | | 60.735 | | 60.768 | | 60.802 |
| Ascorbic acid-type compound | Calcium IPDAA | | 1.500 | | 1.500 | | 1.500 |
| Monomer | 2-HBMA | 1.757 | 1.869 | 1.757 | 1.870 | 1.757 | 1.871 |
| | MDP | 10.000 | | 10.000 | | 10.000 | |
| | TEGDMA | 7.907 | 8.409 | 7.907 | 8.414 | 7.907 | 8.419 |
| | UDMA | 19.550 | 18.688 | 19.550 | 18.698 | 19.550 | 18.708 |
| Organic peroxide | Luperox TAH | 2.000 | | 2.000 | | 2.000 | |
| Photopolymerization initiator | BEDB | | 0.100 | | 0.100 | | 0.100 |
| | CQ | | 0.100 | | 0.100 | | 0.100 |
| Stabilizer | BHT | 0.044 | 0.046 | 0.044 | 0.047 | 0.044 | 0.047 |
| Silanol condensing agent | Ti(OBu)4 | 0.300 | | 0.300 | | 0.300 | |
| Adhesiveness evaluation | Dentin (MPa) | AA | | AA | | AA | |
| | Feldspar ceramics (MPa) | A | | A | | A | |

**[Table 8]**

| | | Example | | | |
|---|---|---|---|---|---|
| | | 18 | | 19 | |
| | | First agent | Second agent | First agent | Second agent |
| Polymerization accelerator | ATH | | 0.150 | | 0.150 |
| | Triphenyl Phosphite | | 1.000 | | 1.000 |
| Transition metal compound | Cu(OAc)2 monohydrate | 0.020 | | 0.020 | |
| Compound A | 8-MOS | | 8.000 | | 6.000 |
| Filler | IS TGF2722A | 57.104 | | 57.104 | |
| | R812 | 1.318 | 1.371 | 1.318 | 1.402 |
| | SG-YBF100WSCMP10 | | 59.402 | | 60.735 |
| Ascorbic acid-type compound | Calcium IPDAA | | 1.500 | | 1.500 |
| Monomer | 2-HBMA | 1.757 | 1.828 | 1.757 | 1.869 |
| | MDP | 10.000 | | 10.000 | |
| | TEGDMA | 7.907 | 8.225 | 7.907 | 8.409 |
| | UDMA | 19.550 | 18.278 | 19.550 | 18.688 |
| Organic peroxide | Luperox TAH | 2.000 | | 2.000 | |
| Photopolymerization initiator | BEDB | | 0.100 | | 0.100 |
| | CQ | | 0.100 | | 0.100 |
| Stabilizer | BHT | 0.044 | 0.045 | 0.044 | 0.046 |
| Silanol condensing agent | Ti(OBu)4 | 0.300 | | 0.300 | |
| Adhesiveness evaluation | Dentin (MPa) | AA | AA | | |
| | Feldspar ceramics (MPa) | A | A | | |

**[Table 9]**

| | | Example | | | | | |
|---|---|---|---|---|---|---|---|
| | | 20 | | 21 | | 22 | |
| | | First agent | Second agent | First agent | Second agent | First agent | Second agent |
| Polymerization accelerator | ATH | | 0.150 | | 0.150 | | 0.150 |
| | DitBuPhenyl-biphenyl-phosphonite | | | | 1.000 | | |
| | Triphenyl Phosphite | | 1.000 | | | | |
| Transition metal compound | Cu(OAc)2 monohydrate | 0.020 | | 0.020 | | 0.020 | |
| Compound A | 8-MOS | | 6.000 | | 6.000 | | 6.000 |
| Filler | IS TGF2722A | 57.104 | | 57.104 | | 57.104 | |
| | R812 | 1.318 | 1.402 | 1.318 | 1.402 | 1.318 | 1.417 |
| | SG-YBF100WSCMP10 | | 60.735 | | 60.735 | | 61.401 |
| Ascorbic acid-type compound | Calcium IPDAA | | 1.500 | | 1.500 | | 1.500 |
| Monomer | 2-HBMA | 1.757 | 1.869 | 1.757 | 1.869 | 1.757 | 1.889 |
| | MDP | 10.000 | | 10.000 | | 10.000 | |
| | TEGDMA | 7.907 | 8.409 | 7.907 | 8.409 | 7.907 | 8.502 |
| | UDMA | 19.550 | 18.688 | 19.550 | 18.688 | 19.550 | 18.893 |
| Organic peroxide | Luperox TAH | 2.000 | | 2.000 | | 2.000 | |
| Photopolymerization initiator | BEDB | | 0.100 | | 0.100 | | 0.100 |
| | CQ | | 0.100 | | 0.100 | | 0.100 |
| Stabilizer | BHT | 0.044 | 0.046 | 0.044 | 0.046 | 0.044 | 0.047 |
| Silanol condensing agent | Ti(OBu)4 | 0.300 | | 0.300 | | 0.300 | |
| Adhesiveness evaluation | Dentin (MPa) | AA | | AA | | AA | |
| | Feldspar ceramics (MPa) | A | | A | | A | |

**[Table 10]**

| | | Example | | | | | | | | | |
|---|---|---|---|---|---|---|---|---|---|---|---|
| | | 23 | | 24 | | 25 | | 26 | | 27 | |
| | | First agent | Second agent | First agent | Second agent | First agent | Second agent | First agent | Second agent | First agent | Second agent |
| Polymerization accelerator | ATH | | 0.150 | | 0.100 | | 0.050 | | 0.025 | | 0.010 |
| Transition metal compound | Cu(OAc)2 monohydrate | 0.020 | | 0.020 | | 0.020 | | 0.020 | | 0.020 | |
| Compound A | 8-MOS | | 6.000 | | 6.000 | | 6.000 | | 6.000 | | 6.000 |
| Filler | IS TGF2722A | 57.104 | | 57.104 | | 57.104 | | 57.104 | | 57.104 | |
| | R812 | 1.318 | 1.417 | 1.318 | 1.418 | 1.318 | 1.419 | 1.318 | 1.419 | 1.318 | 1.419 |
| | SG-YBF100WSCMP10 | | 61.401 | | 61.435 | | 61.468 | | 61.485 | | 61.495 |
| Ascorbic acid-type compound | Calcium IPDAA | | 1.500 | | 1.500 | | 1.500 | | 1.500 | | 1.500 |
| Monomer | 2-HBMA | 1.757 | 1.889 | 1.757 | 1.890 | 1.757 | 1.891 | 1.757 | 1.892 | 1.757 | 1.892 |
| | MDP | 10.000 | | 10.000 | | 10.000 | | 10.000 | | 10.000 | |
| | TEGDMA | 7.907 | 8.502 | 7.907 | 8.506 | 7.907 | 8.511 | 7.907 | 8.513 | 7.907 | 8.515 |
| | UDMA | 19.550 | 18.893 | 19.550 | 18.903 | 19.550 | 18.913 | 19.550 | 18.918 | 19.550 | 18.921 |
| Organic peroxide | Luperox TAH | 2.000 | | 2.000 | | 2.000 | | 2.000 | | 2.000 | |
| Photopolymerization initiator | BEDB | | 0.100 | | 0.100 | | 0.100 | | 0.100 | | 0.100 |
| | CQ | | 0.100 | | 0.100 | | 0.100 | | 0.100 | | 0.100 |
| Stabilizer | BHT | 0.044 | 0.047 | 0.044 | 0.047 | 0.044 | 0.047 | 0.044 | 0.047 | 0.044 | 0.047 |
| Silanol condensing agent | Ti(OBu)4 | 0.300 | | 0.300 | | 0.300 | | 0.300 | | 0.300 | |
| Adhesiveness evaluation | Dentin (MPa) | AA | | AA | | AA | | AA | | AA | |
| | Feldspar ceramics (MPa) | A | | A | | A | | A | | A | |

**[Table 11]**

| | | Example | | | | | | | |
|---|---|---|---|---|---|---|---|---|---|
| | | 28 | | 29 | | 30 | | 31 | |
| | | First agent | Second agent | First agent | Second agent | First agent | Second agent | First agent | Second agent |
| Polymerization accelerator | ATH | | 0.030 | | 0.030 | | 0.030 | | 0.030 |
| | DitBuPhenyl-biphenyl-phosphonite | | 0.600 | | 0.600 | | 0.600 | | 0.600 |
| Transition metal compound | Cu(OAc)2 monohydrate | 0.020 | | 0.020 | | 0.020 | | 0.020 | |
| Compound A | 8-MOS | | 4.680 | | 4.680 | | 4.680 | | 4.680 |
| Filler | IS TGF2722A | 57.104 | | 57.104 | | 57.104 | | 57.104 | |
| | R812 | 1.318 | 4.714 | 1.318 | 4.684 | 1.318 | 4.654 | 1.318 | 4.601 |
| | SG-YBF100WSCMP10 | | 59.814 | | 59.434 | | 59.053 | | 58.387 |
| Ascorbic acid-type compound | Calcium C12AAA | | 0.300 | | 0.900 | | 1.500 | | 2.550 |
| Monomer | 2-HBMA | 1.757 | | 1.757 | | 1.757 | | 1.757 | |
| | 2-HPMA | | 1.885 | | 1.873 | | 1.861 | | 1.840 |
| | MDP | 10.000 | | 10.000 | | 10.000 | | 10.000 | |
| | TEGDMA | 7.907 | 8.484 | 7.907 | 8.430 | 7.907 | 8.376 | 7.907 | 8.282 |
| | UDMA | 19.550 | 19.325 | 19.550 | 19.202 | 19.550 | 19.079 | 19.550 | 18.864 |
| Organic peroxide | Luperox TAH | 2.000 | | 2.000 | | 2.000 | | 2.000 | |
| Photopolymerizatio n initiator | BEDB | | 0.060 | | 0.060 | | 0.060 | | 0.060 |
| | CQ | | 0.060 | | 0.060 | | 0.060 | | 0.060 |
| Stabilizer | BHT | 0.044 | 0.048 | 0.044 | 0.047 | 0.044 | 0.047 | 0.044 | 0.046 |
| Silanol condensing agent | Ti(OBu)4 | 0.300 | | 0.300 | | 0.300 | | 0.300 | |
| Adhesiveness evaluation | Dentin (MPa) | AA | | A | | AA | | AA | |
| | Feldspar ceramics (MPa) | A | | A | | A | | A | |

As shown in each table, in the examples using the curable composition preparation kit including: a first agent containing a first monomer; and a second agent containing a second monomer, in which at least one of the first agent or the second agent each independently contains an ascorbic acid-type compound and a compound A containing at least one of an alkoxysilyl group or a hydroxysilyl group, cured products with excellent adhesiveness to both ceramics and dentin were obtained.

On the other hand, Comparative Example 1 and Comparative Example 2 using the curable composition preparation kit without the ascorbic acid-type compound did not adhere to dentin, and exhibited poor adhesiveness to dentin.

Comparative Example 3 using the curable composition preparation kit without the compound A exhibited poor adhesiveness to ceramics.

The disclosure of Japanese Patent Application No. 2022-124314 filed on August 3, 2022 is incorporated herein by reference in its entirety.

All publications, patent applications, and technical standards mentioned in the present specification are incorporated in the present specification to the same extent as if each individual publication, patent application, and technical standard were specifically and individually noted to be incorporated by reference.

## Claims

1. A curable composition preparation kit, comprising:
a first agent comprising a first monomer; and
a second agent comprising a second monomer,
wherein at least one of the first agent or the second agent each independently comprises an ascorbic acid-type compound and a compound A comprising at least one of an alkoxysilyl group or a hydroxysilyl group.

2. The curable composition preparation kit according to claim 1, wherein the compound A has a molecular weight of 200 or more.

3. The curable composition preparation kit according to claim 1, wherein the compound A comprises at least one selected from the group consisting of:
an organosilane comprising a polymerizable group and an alkoxy group or a hydroxyl group; and
an organosiloxane comprising a polymerizable group, an alkoxy group or a hydroxyl group, and a siloxane bond.

4. The curable composition preparation kit according to claim 1, wherein a content of the compound Ais 50% by mass or more with respect to a total amount of the ascorbic acid-type compound and the compound A.

5. The curable composition preparation kit according to claim 1, wherein at least one of the first agent or the second agent each independently comprises a transition metal compound and an organic peroxide.

6. The curable composition preparation kit according to claim 5, wherein:
the first agent comprises the transition metal compound and the organic peroxide, and
the second agent comprises the ascorbic acid-type compound and the compound A.

7. The curable composition preparation kit according to claim 1, wherein one of the first monomer or the second monomer comprises an acidic monomer.

8. The curable composition preparation kit according to claim 7, wherein the first monomer comprises the acidic monomer, and
the second agent comprises the ascorbic acid-type compound and the compound A.

9. The curable composition preparation kit according to claim 1, wherein at least one of the first agent or the second agent comprises at least one polymerization accelerator (1) selected from the group consisting of a phosphonite compound, a phosphite compound, and a sulfite compound.

10. The curable composition preparation kit according to claim 1, wherein at least one of the first agent or the second agent comprises a polymerization accelerator (2) that is a polymerization accelerator other than at least one polymerization accelerator (1) selected from the group consisting of a phosphonite compound, a phosphite compound, and a sulfite compound.

11. The curable composition preparation kit according to claim 1, wherein at least one of the first agent or the second agent comprises a silanol condensing agent.

12. The curable composition preparation kit according to claim 1, wherein the kit is for a dental material.

13. A curable composition which is a mixture of the first agent and the second agent in the curable composition preparation kit according to claim 1 or 2.

14. A cured product of the curable composition according to claim 13.

15. A dental material comprising the cured product according to claim 14.
